(19) Europäisches Patentamt
European Patent Office
Office européen des brevets

(11) **EP 4 297 629 B1**

(12) **EUROPEAN PATENT SPECIFICATION**

(45) Date of publication and mention of the grant of the patent:
**11.02.2026 Bulletin 2026/07**

(21) Application number: **22710357.9**

(22) Date of filing: **25.02.2022**

(51) International Patent Classification (IPC):
***A61B 3/06*** *(2006.01)*

(52) Cooperative Patent Classification (CPC):
**A61B 3/063**

(86) International application number:
**PCT/EP2022/054877**

(87) International publication number:
**WO 2022/180246 (01.09.2022 Gazette 2022/35)**

# (54) METHOD FOR DETERMINING AT LEAST ONE OPTICAL PRODUCT INTENDED TO FACE AN EYE OF A USER USING AN EYE RESISTANCE LEVEL AND A LIGHT PROTECTION LEVEL

VERFAHREN ZUR BESTIMMUNG VON MINDESTENS EINEM OPTISCHEN PRODUKT ZUR GESICHTUNG EINES AUGES EINES BENUTZERS UNTER VERWENDUNG EINER AUGENWIDERSTANDSEBENE UND EINER LICHTSCHUTZEBENE

PROCÉDÉ DE DÉTERMINATION D'AU MOINS UN PRODUIT OPTIQUE DESTINÉ À FAIRE FACE À L'OEIL D'UN UTILISATEUR UTILISANT UN NIVEAU DE RÉSISTANCE OCULAIRE ET D'UN NIVEAU DE PROTECTION CONTRE LA LUMIÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2021 PCT/IB2021/000125**
**31.05.2021 PCT/IB2021/000401**

(43) Date of publication of application:
**03.01.2024 Bulletin 2024/01**

(73) Proprietor: **Essilor International**
**94220 Charenton-Le-Pont (FR)**

(72) Inventors:
- **MARIE, Sarah**
  **94220 CHARENTON-LE-PONT (FR)**
- **SCHERLEN, Anne-Catherine**
  **75016 PARIS (FR)**
- **BARRAU, Coralie**
  **75012 PARIS (FR)**
- **POPHILLAT, Olivier**
  **94520 PERIGNY SUR YERRES (FR)**
- **MAURY, Hélène**
  **75011 PARIS (FR)**
- **DUMONT, Jean-Philippe**
  **75019 PARIS (FR)**
- **EHRISMANN, Camille**
  **49080 BOUCHEMAINE (FR)**
- **CASTILLO LLAVE, Mayara**
  **TUAM, H54 - RD 25 (IE)**
- **DOUSSINAULT, Cécile**
  **DUBLIN, 2 (IE)**

(74) Representative: **Ipsilon**
**12 Avenue d'Italie**
**75013 Paris (FR)**

(56) References cited:
**EP-A1- 3 753 475** **WO-A1-2021/102169**
**US-A- 6 099 126** **US-A1- 2019 212 581**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 297 629 B1

## Description

The present invention generally relates to the field of light sensitivity.

It more particularly relates to a method for determining a visual discomfort and/or a visual performance of a user as well as a method for determining at least one filter for a transparent support able to improve or to maintain the visual comfort and/or visual performance of a user.

It also relates to a computer system and a computer program product comprising code instructions for performing said methods.

It has been observed that most people, close to 9/10 people, experience sensitivity to light. It can be a sensation of discomfort, headache, pain, dazzling, or fatigue. It is a visual discomfort which can occur in many lighting conditions, notably in lighting conditions specific for a given user.

It is known to correlate a light sensitivity to a light intensity. In this regard, it is possible to determine for a user a light sensitivity threshold at which a light exposure becomes uncomfortable.

However, it is difficult to evaluate such light sensitivity in a global manner. Indeed, a user may be exposed to different kinds of lights in his everyday life and may have a light sensitivity which varies with these kinds of lights. These light exposures are for example a warm light, a cold light or a blinking light.

As an example, a user may be very sensitive to cold light or blinking light but less sensitive to warm light. Another user may have a low sensitivity to warm and cold light but a high sensitivity to blinking light. It could thus be very difficult, notably for an eye care professional (ECP) or for the user himself, to accurately evaluate the light sensitivity of this user in a global manner. Particularly, it is difficult to easily obtain a meaningful quantity or value representative of the global light sensitivity of the user.

Furthermore, directly combining these different light sensitivity thresholds do not allow to obtain a usable data actually representative of the light sensitivity of the user. Indeed, these data do not refer to the same luminance, to the same light conditions and/or the same nature of threshold. Combining these data is purely theoretical and do not provide usable data.

EP 3 753 475 discloses a method for determining a filter for a transparent support to improve visual comfort/-performance of a user. It determines a light sensitivity threshold by exposing the user to different light conditions (warm light, cold light, and flashing light), and determines an index representative of protection required based on light environments. The method determines a score for filters and light environments to find the best filter for the user.

US 6,099,126 discloses a system for testing light sensitivity, exposing a user to varying light intensities while viewing images, and recording when the user indicates discomfort. It suggests comparing results to a scale developed from multiple subjects.

US 2019/212581 discloses determining visual discomfort by measuring a subject's threshold of light sensitivity, which corresponds to the light intensity at which visual discomfort is expressed by the wearer. The document explains that a user's comfort level can be quantified using a subjective comfort indicator on a standardized evaluation scale. It also discusses the relationship between light sensitivity thresholds and determining appropriate filters with specific transmission properties.

A problem that the invention aims to solve is thus to provide a parameter representative of a visual discomfort of a user with regard to different light conditions, this parameter allowing to compare light sensitivity of different users with each other.

To solve this problem, the invention provides a method for determining a visual discomfort and/or a visual performance of a user, the method being defined in claim 1 and comprising the following steps:

- providing at least two quantities representative of a light sensitivity threshold of the user;

- determining a resistance level based on said at least two quantities representative of a light sensitivity threshold of the user using a percentile scale based on a population baseline.

The eye resistance level is the capacity of the eye to manage the light intensity through photoreceptor and/or cortical processing, i.e. before the photoreceptors are saturated. Therefore, the eye resistance level is a global parameter which is able to evaluate the ability of a user to withstand light exposure.

Determining said eye resistance level depending of quantities representative of a light sensitivity threshold allows to enhance the accuracy and robustness of this parameter.

Using a percentile scale based on a population baseline allows to make it possible to use a same reference to gather these quantities representative of a light sensitivity threshold. When these quantities are expressed using a same reference, it is meaningful to combine thereof to determine said eye resistance level. As such, it is possible to obtain a parameter which depends on quantities representative of a light sensitivity threshold which may be obtained using different light conditions. For example, these light conditions can be a warm light exposure, a cold light exposure or a blinking light exposure.

The present method allows to combine different information related to the light sensitivity of the user, for example for different light conditions, by using a same comparable reference scale.

According to an embodiment of the determining method, the step of determining said resistance level comprises:

- determining, for each quantity of said at least two quantities representative of a light sensitivity threshold of the user, a percentage value corresponding to the quantity representative of a light sensitivity threshold of the user in said percentile scale,
- determining said resistance level depending on said percentage values.

According to an embodiment of the determining method, said resistance level is determined as a combination of said percentage values.

According to an embodiment of the determining method, said percentage values are determined using a same percentile scale.

Said at least two quantities representative of a light sensitivity threshold of the user are determined by exposing the user to different light conditions.

According to an embodiment of the determining method, said at least two quantities representative of a light sensitivity threshold of the user comprise a first and a second quantities respectively representative of a low and a high light sensitivity threshold of the user, said high sensitivity threshold corresponding to a discomfort of the user greater than the low sensitivity threshold.

According to an embodiment of the determining method, said at least two quantities representative of a light sensitivity threshold of the user is at least one among: a warm light sensitivity threshold, a cold light sensitivity threshold and a blinking light sensitivity threshold.

According to an embodiment of the determining method, the step of determining said resistance level comprises:

- providing a plurality of resistance level groups based on a resistance level distribution of said population baseline,
- determining a resistance level group corresponding to the user depending on said resistance level.

According to an embodiment of the determining method, it further comprises the step of determining at least one filter for a transparent support able to improve or to maintain the visual comfort and/or visual performance of a user based on said determined resistance level.

According to an embodiment of the determining method, a first resistance level is determined based on at least two quantities representative of a light sensitivity threshold of a first user using a percentile scale based on a first population baseline, said method further comprising;

- determining an updated population baseline based on said at least two quantities representative of a light sensitivity threshold of a first user and/or said first resistance level,
- determining a second resistance level based on at least two quantities representative of a light sensitivity threshold of a second user using a percentile scale based on said updated population baseline.

The invention also relates to a method for determining at least one filter for a transparent support able to improve or to maintain the visual comfort and/or visual performance of a user, the method comprising the following steps:

- determining a resistance level using the method as described above,
- determining, for each light environment among a group of light environments, an index representative of the level of protection required by the user;
- determining a score for each light environment among the group of light environments and for each filter among a group of filters, said score being representative of the capacity of the filter to reach the level of protection required by the user;
- determining at least one filter among the group of filters based on the scores of said at least one filter in a plurality of light environments among the group of light environments.

According to an embodiment of the determining method, it further comprises a step of comparing said at least one quantity representative of a light sensitivity threshold of the user to a sensitivity reference to determine if the user is slightly sensitive or non-sensitive.

According to an embodiment of the determining method, said index representative of the level of protection required by the user is determined using a different questionnaire if the user is determined as a slightly sensitive or non-sensitive user.

The invention also relates to a method for determining at least one optical product intended to face an eye of a user, the method comprising the following steps:

- determining at least one light protection need of a user by performing the method for determining a visual discomfort and/or a visual performance of a user as previously described,
- determining at least one short-term light protection score representative of at least one short-term light protection attribute of said at least one optical product,
- determining at least one long-term light protection score representative of at least one long-term light protection attribute of said at least one optical product,
- evaluating at least one light protection level of at least one optical product based on said at least one short-term and at least one long-term protection scores,

determining at least one optical product for the user based on said at least one light protection level and said at least one light protection need of the user.

The light protection level is thus evaluated considering both short-term and long-term protections to allow a more complete evaluation. The combination of short-term and long-term protections allows to make the light protection level more relevant to much more light environments and conditions. It better reflects the light protection level of the optical product in a holistic and ecological approach.

It is thus easier for an ECP or a user to determine which optical product is adapted to the user's light protection needs.

According to an embodiment of the determining method, the light sensitivity threshold is determined using a device configured to expose the user to an increasing light level and to determine the at least one quantity representative of the user's light sensitivity threshold based on a user's feedback representative of a discomfort.

According to an embodiment of the determining method, it further comprises:

- determining at least one light environment,
- selecting said at least one short-term light protection attribute and said at least one long-term light protection attribute respectively among a group of short-term light protection attributes and a group of long-term light protection attributes, depending on said at least one determined light environment.

According to an embodiment of the determining method, said at least one light environment are selected within a group of light environments, said group of light environments comprises at least one among a bright light environment, a day drive environment, a night drive environment, an indoor environment, a screen at night environment, an in-to-out transition environment and an out-to-in transition environment.

According to an embodiment of the determining method, said at least one light environment is selected depending on at least one subjective and/or objective data from the user.

According to an embodiment of the determining method, said subjective and/or objective data from the user are at least one among a user's choice, habits of the user, data from a questionnaire fulfilled by the user

According to an embodiment of the determining method, said at least one short-term and/or said at least one long-term light protection scores are determined using different weightings for said at least one short-term and said at least one long-term light protection attributes, respectively.

According to an embodiment of the determining method, it further comprises a step of providing at least three light protection categories, the step of determining said at least one short-term and said at least one long-term light protection scores comprising a step of identifying a light protection category corresponding to the said at least one short-term and said at least one long-term light protection scores.

According to an embodiment of the determining method, said at least three light protection categories are determined to correspond to said resistance level groups so that when a resistance level group is determined, the corresponding light protection category is identified as a light protection threshold for the user.

According to an embodiment of the determining method, said at least one short-term light protection attribute is a transmission attribute (Tv) and said at least one long-term attribute is a blue radiation filtering (BVC(B')).

According to an embodiment of the determining method, the light protection categories for the transmission attribute (Tv) are the following:

- first light protection category: at least 95%,
- second light protection category: between 91% and 95%,
- third light protection category: between 85% and 90%;

and wherein the light protection categories for the blue radiation filtering (BVC(B')) are the following:

- first light protection category: between 11 and 19%,
- second light protection category: between 20% and 34%,
- third light protection category: between 35% and 79%.

According to an embodiment of the determining method, it further comprises:

- determining at least one vision experience score representative of at least one optical product quality attribute of said at least one optical product,
- determining at least one global short-term light protection score based on said at least one short-term light protection score and said at least one vision experience score,

wherein said at least one light protection level of said at least one optical product is evaluated based on said at least one global short-term light protection score and said at least one long-term light protection score.

According to an embodiment of the determining method, the step of evaluating at least one protection level comprises a step of determining at least one global light protection score of said at least one optical product based on said at least one short-term and at least one long-term light protection scores.

According to an embodiment of the determining method, it further comprises:

- determining at least two light environments,

wherein at least one short-term, at least one long-term and at least one global light protection scores are determined for each determined light environment.

According to an embodiment of the determining method, it further comprises the step of determining a light protection evaluation score based on each global light protection score determined for a determined light environment, wherein said light protection evaluation score is determined using different weightings for said global light protection scores depending on the at least two determined light environments.

According to an embodiment of the determining method, said global light protection score is determined using different weightings for said at least one global short-term and said at least one long-term light protection scores.

According to an embodiment of the determining method, said at least one short-term light protection attribute is one among a transmission attribute, a polarization attribute, a stray light attribute and a spectral attribute.

According to an embodiment of the determining method, wherein said at least one long-term attribute is a spectral attribute.

According to an embodiment of the determining method, it further comprises a step of displaying said at least one short-term and said at least one long-term light protection scores onto a same graphic having a first and a second dimensions, said at least one short-term light protection being positioned relative to said first dimension and said at least one long-term light protection being positioned relative to said second dimension.

The method is a computer-implemented method.

The invention also provides a computer system for determining a visual discomfort and/or a visual performance of a user, the system being defined in claim 13 and comprising:

- a processor; and
- a memory with computer code instructions stored thereon, the memory operatively coupled to the processor such that, when executed by the processor, the computer code instructions cause the computer system to perform a method for determining at least one optical product intended to face an eye of a user, the method comprising the following steps:
- determining at least one light protection need of a user by:

  - providing at least two quantities representative of a light sensitivity threshold of the user;
  - determining a resistance level based on said at least two quantities representative of a light sensitivity threshold of the user using a percentile scale based on a population baseline,

- determining at least one short-term light protection score representative of at least one short-term light protection attribute of said at least one optical product,
- determining at least one long-term light protection score representative of at least one long-term light protection attribute of said at least one optical product,
- evaluating at least one light protection level of at least one optical product based on said at least one short-term and at least one long-term protection scores.

The invention further provides an optical product comprising a frame and at least one optical system attached to said frame and intended to face an eye of a user, said optical product comprising a data collecting device and a controller configured to perform a method for determining at least one optical product intended to face an eye of a user, the method comprising the following steps:

- determining at least one light protection need of a user by:

  - providing at least two quantities representative of a light sensitivity threshold of the user;
  - determining a resistance level based on said at least two quantities representative of a light sensitivity threshold of the user using a percentile scale based on a population baseline,

- determining at least one short-term light protection score representative of at least one short-term light protection attribute of said at least one optical product,
- determining at least one long-term light protection score representative of at least one long-term light protection attribute of said at least one optical product,
- evaluating at least one light protection level of at least one optical product based on said at least one short-term and at least one long-term protection scores,
- determining at least one optical product for the user based on said at least one light protection level and said at least one light protection need of the user.

The invention is described in more detail below by way of the figures that show only one preferred embodiment of the invention.

Figure 1 schematically shows a graph of a plurality of sets of initial data expressed in a percentile scale based on a population baseline.
Figure 2 schematically shows a flowchart of a filter determining method.
Figure 3 schematically shows a light sensitivity test performed using a dedicated device.
Figures 4 to 6 schematically shows a data restitution regarding the results of the light sensitivity test displayed on displaying device for respectively warm light, cold light and blinking light conditions.
Figure 7, schematically shows a first questionnaire for sensitive or highly sensitive users.
Figure 8 schematically shows a second questionnaire for non-sensitive or slightly sensitive users.
Figure 9 shows a flowchart of a score determining step.
Figure 10 schematically shows a data restitution regarding the results of the questionnaire displayed on a displaying device.
Figure 11 shows schematically a data restitution of a filter determining step displayed on displaying device.
Figure 12 shows a flowchart of a method according to the invention evaluating a light protection level of an optical product.
Figure 13 shows a table gathering weighting factors of optical product quality attributes used in the evaluating method of figure 12.
Figure 14 shows a table gathering an example of short-term light protection, long-term light protection and optical product quality attributes considered to determine a light protection level according to said method of figure 12 for each selected light environment.
Figure 15 shows a table gathering an example of weighting factors applied to short-term light protection, long-term light protection and optical product quality attributes for each selected light environment.
Figure 16 shows a table gathering technical values and associated scores of short-term light protection, long-term light protection and optical product quality attributes for a given light environment for a plurality of optical products.
Figure 17 shows a table gathering short-term light protection score, long-term light protection score, vision experience light protection score, global short-term light protection score and global light protection score for each of the optical products of figure 5.
Figure 18 shows a graph illustrating the global light protection score of each of the optical products of figures 16 and 17.
Figure 19 shows a graph wherein each optical product of figures 16 and 17 is represented by a point having a X-axis coordinate referring to the global short-term light protection score and a y-axis coordinate referring to the long-term light protection score.

In the description which follows, the drawing figures are not necessarily to scale and certain features may be shown in generalized or schematic form in the interest of clarity and conciseness or for informational purposes. In addition, although making and using various embodiments are discussed in detail below, it should be appreciated that as described herein are provided many inventive concepts that may embodied in a wide variety of contexts. Embodiments discussed herein are merely representative and do not limit the scope of the invention. It will also be obvious to one skilled in the art that all the technical features that are defined relative to a process can be transposed, individually or in combination, to a device and conversely, all the technical features relative to a device can be transposed, individually or in combination, to a process.

The terms "comprise" (and any grammatical variation thereof, such as "comprises" and "comprising"), "have" (and any grammatical variation thereof, such as "has" and "having"), "contain" (and any grammatical variation thereof, such as "contains" and "containing"), and "include" (and any grammatical variation thereof such as "includes" and "including") are open-ended linking verbs. They are used to specify the presence of stated features, integers, steps or components or groups thereof, but do not preclude the presence or addition of one or more other features, integers, steps or components or groups thereof. As a result, a method, or a step in a method, that "comprises", "has", "contains", or "includes" one or more steps or elements possesses those one or more steps or elements but is not limited to possessing only those one or more steps or elements.

The present invention provides a method for determining a visual discomfort and/or a visual performance of a user. To this end, a specific physiologic parameter is determined: the eye resistance level.

The resistance level is the capacity of the eye to manage the light intensity through photoreceptor and/or cortical processing. If the light exposure is lower to the eye resistance level, the visual processing is optimal on the retina, i.e. the comfort and vision are optimal due to a quick adaptation. If the light exposure is higher to the eye resistance level, the photoreceptors are saturated (Mainster MA, Turner PL. Glare's causes, consequences, and clinical challenges after a century of ophthalmic study. American journal of ophthalmology. 2012;153(4):587-593 and Efalov VJ. Rod and cone visual pigments and phototransduction through pharmacological, genetic, and physiological approaches. J Biol Chem. 2012;287(3):1635-1641*)*, neural hyperexcitability appears (Mainster MA, Turner PL. Glare's causes, consequences, and clinical challenges after a century of ophthalmic study. American journal of ophthalmology. 2012;153(4):587-593*)*, inducing discomfort, disability and pain for the user (Noseda R, Kainz V, Jakubowski M, et al. A neural mechanism for exacerbation of headache by light. Nat Neurosci. 2010;13(2):239-245. doi:10.1038/nn.2475*)*.

Eye resistance level thus refers to a limit before an over light exposure inducing a saturation of the photoreceptors causing discomfort, pain.

This method comprises the provision of a plurality of quantities representative of a light sensitivity threshold of the user. These quantities are preferably an illuminance expressed in lux.

By "sensitivity to light" of the user, what is meant is any relatively intense and prolonged reaction or modification of comfort or visual performance in relation to a temporary or continuous light flux or stimuli.

The light sensitivity threshold may be determined based on measurements using a dedicated device. This dedicated device may be a device configured to emit light toward one or both eyes of the user. The light sensitivity threshold may be determined depending on a response provided by the user subsequently to the light exposure. This response may be intentional by asking the user to indicate when a discomfort very disturbing occurs or determined by an external device configured to detect physical response to the user to a given light flux. In both cases, the quantity representative of a light sensitivity threshold of the user may be determined as a radiometric parameter or a photometric parameter.

A photometric parameter may be the illuminance (Lux) which is to a luminous flux incident on a surface (e.g. the front of the eyes) or the luminance ($cd/m^2$) which is a luminous flux per unit solid angle per unit projected source area. A photometric parameter may also be the luminous exposure (Lux second) which is the time-integrated illuminance.

A radiometric parameter is the energy of the eye without taking into account eye sensitivity to wavelength (visibility). Said radiometric parameter may be the irradiance or flux density (Watt per square meter, i.e. $W/m^2$) which is the radiant flux received by a surface per unit area. This is sometimes also confusingly called "intensity". Said radiometric parameter is equivalent to Illuminance in radiometry. Said radiometric parameter may also be the spectral irradiance or spectral flux density (Watt per square meter, per meter, i.e. $W/m^3$) which is the irradiance on a limited range of wavelength, e.g. only blue light. Said radiometric parameter may be the radiance (Watt per steradian per square meter, i.e. $W.sr^{-1}.m^{-2}$) which is the radiant flux emitted, reflected, transmitted or received by a surface, per unit solid angle per unit projected area. Radiance is equivalent to Luminance in radiometry. Said radiometric parameter may also be the spectral radiance (Watt per steradian per square meter, per meter, i.e. $W.sr^{-1}.m^{-3}$) which is the radiance of a surface per unit of wavelength. Alternatively, said radiometric parameter may be a number of photon reaching the eye per second on all visible spectrum or a limited part of the spectrum or the troland ($cd/m^2.mm^2$) which is the luminance weighted by pupil size.

The light sensitivity threshold may be set to define different levels or natures of discomfort of the user. In other words, the level or nature of the light sensitivity threshold may vary among the plurality of quantities representative of a light sensitivity threshold of the user. Hence, said plurality of quantities representative of a light sensitivity threshold of the user mays comprise a first quantity representative of a first light sensitivity threshold of the user and a second quantity representative of a second light sensitivity threshold of the user.

A variation of the nature or level of the light sensitivity threshold preferably refers to different symptoms of the user. As an example, the light sensitivity threshold may identify a just perceptible discomfort or a very disturbing discomfort. A just perceptible discomfort may refer to a start of tension in the eyelids of the user or tingling in the eyes. A very disturbing discomfort may refer to a moment when a significant effort is required to keep the eyes open.

In a preferred embodiment, said plurality of quantities representative of a light sensitivity threshold refer to different levels of discomfort of the user. In other words, said at least two quantities representative of a light sensitivity threshold of the user may comprise a first and a second quantities respectively representative of a low and a high light sensitivity threshold of the user, said high sensitivity threshold corresponding to a discomfort of the user greater than the low sensitivity threshold. The low light sensitivity threshold may refer to a just perceptible discomfort and the high light sensitivity threshold may refer to a very disturbing discomfort. More generally, the different light sensitivity thresholds may comprise a low, at least one intermediate and a high light sensitivity thresholds.

Determining the resistance level based on different levels of discomfort of the user allows to obtain a more detailed view of the light discomfort of the user. Indeed, the user may experience an early starting discomfort but a very late disturbing discomfort. On the contrary, another user may experience very close just perceptible and very disturbing discomforts. Considering different levels of light sensitivity threshold allows to better reflect the sensitivity of the user.

Furthermore, quantities representative of a light sensitivity threshold may be determined by exposing the user to different light conditions or environments.

It has been observed that a light environment cannot be accurately defined by only considering a single parameter as light intensity, this light environment is dynamic and composed of a plurality of components allowing to better describe thereof. Light may be defined as comprising at least four main components (called "4D"): an intensity component, a spatial component, a temporal component and a spectral component.

The intensity component refers to the luminous flux emitted by a light source in Lumen. The intensity component induces an illuminance at the wearer's position expressed in Lux. The illuminance may be determined using a light sensor disposed at a user's eye position which measures the illuminance in Lux induced by a light source. Some of the factors affecting the illuminance are the energetic intensity of a light source (the initial volume), the distance between the user and the source (the volume at any point along a light path) and any modifying elements in the light path (air, clouds, filters, reflectors, etc.). For example, the amount of outdoor light a person is exposed to can vary depending on geographical location, season, time of day, local weather, etc. The intensity component may refer to any one of the radiometric and photometric parameters previously mentioned.

The spatial component is the relative position of the light source regarding the user. This relative position depends on the angular distance between the user and the light source. The spatial component can be punctual or wide, and it can affect the individual's perception of light. Thus, for a given radiant flux, a punctual light source will have a higher luminance due to its smaller size. For example, vehicle headlights of similar radiant flux differ in luminance according to their size and distance.

The temporal component defines the period during which the light source emits. Indeed, light may be present for a short or long period of time, which may change the perception of the light by the user. Exposure to light for a given duration may be continuous or intermittent (which may also vary in frequency). In other words, the user may be exposed to a blinking light. For instance, car headlamps may only contribute to the light environment for a few seconds and move constantly across the field of vision.

The spectral component refers to the spectrum of the light which is emitted and its associated energy. This spectrum may be expressed using the wavelength of the light flux in nanometers. As an example, the visible spectrum of the light is comprised between 380 nm and 780 nm. A light flux of white light may appear warmer or colder depending on its spectrum. The spectral component may also be directly expressed as a color of light, e.g. a cold or warm white light. Emitting light reflecting a cold or warm light, e.g. by emitting more blue light or more red light, allows to respectively simulate substantially artificial or natural light or different light ambiance. Varying the color of light emitted toward the user allows to vary the light spectrum. Light sensitivity of the user can thus be determined with regard to a variation of the spectral component of the light.

At least two of these components may be combined to obtain specific light conditions representative of a predetermined environment, e.g. an environment representative of a frequent daily situation that can be a source of discomfort for the user.

Said resistance level is then determined based on said at least two quantities representative of a light sensitivity threshold of the user. The resistance level is determined using a percentile scale based on a population baseline.

A "percentile scale" is a scale wherein the distribution of ordered values is partitioned into 100 intervals containing the same number of data (100-order quantiles).

The "population baseline" is a plurality of initial data representative of light sensitivity threshold of a set of initial users. By "initial users" we mean users which participate to a similar determination of quantities representative to light sensitivity threshold before the present method is performed on said user. In other words, initial users provide objective and/or subjective data allowing to build-up a reference scale expressed as a percentile scale.

A set of initial data is preferably provided for each quantity representative of a light sensitivity of the user. In doing so, when a first quantity representative of a light sensitivity threshold of the user is determined using first light conditions, a first set of initial data obtained using said first light conditions is provided. This first set of initial data comprises a plurality of quantities representative of a light threshold of the initial users. Using the same light conditions for each of the set of initial data and the quantity representative of a light sensitivity threshold of the user allows to compare thereof using the same conditions of experiment.

In a preferred embodiment, the population baseline comprises at least two sets of initial data respectively corresponding to said at least two quantities of a light sensitivity threshold of the user.

An example of a graph 30 of a plurality of sets of initial data expressed in a percentile scale based on a population baseline is shown on figure 1.

Said population baseline comprises six sets of initial data: a first set of initial data 10, a second set of initial data 12, a third set of initial data 14, a fourth set of initial data 16, a fifth set of initial data 18, a sixth set of initial data 20. Each of these sets of initial data correspond to specific experimental conditions. A curve is illustrated for each of these sets of initial data.

The first set of initial data 10 corresponds to a continuous warm light exposure wherein the light sensitivity threshold refers to a just perceptible (JP) discomfort of the initial users.

The second set of initial data 12 corresponds to a continuous warm light exposure wherein the light sensitivity threshold refers to a very disturbing (VD) discomfort of the initial users.

The third set of initial data 14 corresponds to a continuous cold light exposure wherein the light sensitivity threshold refers to a just perceptible (JP) discomfort of the initial users.

The fourth set of initial data 16 corresponds to a continuous cold light exposure wherein the light sensitivity threshold refers to a very disturbing (VD) discomfort of the initial users.

The fifth set of initial data 18 corresponds to a blinking warm light exposure wherein the light sensitivity threshold refers to a just perceptible (JP) discomfort of the initial users.

The sixth set of initial data 20 corresponds to a blinking warm light exposure wherein the light sensitivity threshold refers to a very disturbing (VD) discomfort of the initial users.

These sets of initial data are in a system having a logarithmic x-axis representing the illuminance 24 in Lux and y-axis representing the percentile scale 22 in percentage.

Said graph 30 should be used as follows: a quantity representative of a just perceptible discomfort of the user obtained with a continuous warm light exposure is 300Lux corresponds to 30%. It means that 70% of the initial users of the first set of initial data 10 have experienced a just perceptible discomfort at a higher illuminance. As another way to express this result, the light sensitivity of the user belongs to the second decile of the population baseline corresponding to the first set of initial data 10.

When determining the resistance level, a percentage value corresponding to the quantity representative of a light sensitivity threshold of the user in said percentile scale is determined for each quantity of said at least two quantities representative of a light sensitivity threshold of the user. In the example of the graph 30, each quantity representative of a light sensitivity threshold of the user is correlated to a corresponding curve to obtain a percentage value.

Said resistance level is then determined depending on said percentage values. Particularly, said resistance level is determined as a combination of said percentage values. This combination may be a mean of these percentage values. A ponderation may also be applied between these quantities, e.g. because of precision of one measurement to another or to taking into account lifestyle.

We can take as an example a first quantity representative of a just perceptible discomfort of the user obtained with a continuous warm light exposure and a second quantity representative of a very disturbing discomfort of the user obtained with a continuous warm light exposure. The first quantity is 300Lux and the second quantity is 500Lux. Using the graph 30 of figure 1, the first percentage value (JP) corresponding to said first quantity is 30% and the second percentage value (VD) corresponding to said second quantity is 20%. The resistance level of the user may be determined as the exact mean between these first (JP) and second (VD) percentage values, i.e. 25%. In an alternative way, a ponderation may be applied to the second percentage value to obtain a resistance level more representative of a very disturbing discomfort. If this ponderation is 2 for the second percentage value, we obtain a resistance level of 27%. The ponderation could also be related to a set of initial data which corresponds to a light exposure which is more frequent for the user and therefore more relevant for determining an ophthalmic product.

This resistance level may be determined as a percentage value, a score or a group. Hence, when the resistance level expressed as a percentage value is 25%, said resistance level expressed as a score may be 2/10 and expressed as a group may be "Low resistance level". Expressing said resistance level as a score or a group makes it easier to figure it out for a user.

Said determining method may comprise the provision of a plurality of resistance level groups based on a resistance level distribution of said population baseline. As an example, we can see on figure 1 that three groups are determined: "low resistance" between 0 and 30% corresponding to a score between 1 and 3, "medium resistance" between 31% and 70% corresponding to a score between 4 and 7 and "high resistance" between 71% and 100% corresponding to a score between 8 and 10. A resistance level group may be determined depending on said resistance level.

A filter determining method is further provided to determine at least one filter which fulfills the level of protection required by a user based on an improved determination of the user's sensitivity of light. This filter determining method comprises the above-mentioned resistance level determining method.

By "transparent support", we mean any support through which light may pass and onto which a filter can be disposed to modulate light transmission. The transparent support may be any support intended to be disposed on or in front of an eye of the user. Furthermore, the transparent support may be an ophthalmic lens, a lighting device, an illuminated display, a windshield, a head-mounted display (called "HMD"), a glass, a glass of a portable terminal, etc.

By "*filter*", we mean any means able to modulate light, particularly at least one component of the light (see "4D" below). The filter may be a filter coating or a filtering function which can be used to provide a filter coating. The filter may be in the form of a passive filter (uniform, with a gradient or with a spatial variation) or an active filter as photochromic or electrochromic filters.

The level of protection needed by a user can be better defined when considering a determining method which takes into account all these light components to define light environments. The contribution of this definition of the light in the filter determining method is detailed later in this description.

As shown on figure 2, the filter determining method comprises a step 100 of determining a quantity representative of a light sensitivity threshold of the user, a step 200 of determining an index representative of the level of protection required by the user for specific light environments, a step 300 of determining a score for each light environment and for a plurality of filters and a step 400 of determining at least one filter based on the scores determined at step 300.

The light sensitivity determining step 100 comprises the determination of a quantity representative of a light sensitivity threshold of the user. This quantity is preferably an illuminance expressed in lux.

The light sensitivity threshold may be determined based on measurements using a dedicated device. This dedicated device may be device 10 configured to emit light toward one or both eyes of the user, as shown on figure 3. The device 10 is configured to expose the user to an increasing/decreasing light level and to determine the user's light sensitivity threshold based on a user's feedback representative of a discomfort. The light intensity is increased or decreased to form a light varying sequence. Preferably, this sequence comprises increasing light intensity so as to start light emission with a comfortable intensity for the user. The light sensitivity threshold is then determined depending on a response provided by the user. This response may be intentional by asking the user to indicate when a discomfort very disturbing occurs or determined by an external device configured to detect physical response to the user to a given light flux. In both cases, the quantity representative of a light sensitivity threshold of the user may be determined as the illuminance for which a response of the user is detected.

Furthermore, the light sensitivity threshold may be determined for different colors of light to obtain a threshold reflecting cold or warm white light. Emitting light reflecting a cold or warm light, e.g. by emitting more blue light or more red light, allows to respectively simulate substantially artificial or natural light or different light ambiance. Varying light spectrum through modulation of relative proportion various wavelength allows perceived variation of color (and stimulate differently photoreceptors on the retina). Light sensitivity of the user can thus be determined with regard to a variation of the spectral component of the light.

This light sensitivity determining step 100 is for example performed as follows. The device 10 is disposed in front of the eyes of the user and a light source emits a light flux toward the eyes of the user. A measurement sequence is performed comprising three measurement steps. The first measurement step is a continuous light emission to induce an illuminance from a minimum to a maximum values increasing the illuminance by stages, e.g. from 25 Lux to 10211 Lux. For example, the light emission may start with an illuminance of 25 Lux for 5 seconds to adapt the eye to the light condition and cancel all previous light exposure before the measurement and then continue with an increase of the illuminance of 20% each second to the maximum illuminance. In a more general way, the light may be emitted to induce an illuminance varying from 25 Lux to 15000Lux. This first measurement step is performed with warm light.

The second measurement step is performed identically to the first measurement step but with cold light.

Then, the third measurement step is a flashing light emission to induce an illuminance from a minimum value to a maximum value increasing the illuminance by stages, e.g. from 25 Lux to 8509 Lux. The illuminance of the flashing light emission is preferably increased by at least 20%, preferably by 40%, most preferably by at least 44%. Before and between each flash light emission, the user is subjected to a light emission lower than the minimum value of illuminance of the flashing light emission, e.g. 10 Lux. The time of each flashing light emission is preferably 0,5s and the time between each flashing light emission is preferably 2s.

According to a preferred embodiment, at least one of the first, second and third measurement steps is performed to determine the light sensitivity threshold of the user. A quantity representative of the light sensitivity threshold of the user is thus determined on the basis of the results obtained with at least one of said first, second and third measurement steps. Regarding the definition of light described above, this light sensitivity determining step 100 allows to determine a quantity representative of the light sensitivity threshold with regard to a variation of the intensity, the spatial, the temporal and the spectral components of the light. A global interpretation of the light sensitivity profile of the user may be defined to make the user correspond to a predetermined light sensitivity category, for example from multisensitive to non-sensitive or slighty sensitive. This light sensitivity category is preferably a resistance level group as defined above. As can be seen on figures 4 to 6, these resistance level groups may be "low resistance", "medium resistance" and "high resistance". A high resistance level means that your retina is in good shape. A low resistance level means that you would need more protection in bright conditions to be comfortable.

As shown on figures 4 to 6, results obtained during the light sensitivity determining step 100 may be displayed on a displaying device, as a screen of the computer system. Particularly, these results may comprise at least one light sensitivity threshold determined in at least one of said first, second and third measurement steps. Preferably, each light sensitivity threshold is shown compared to a population baseline to allow the user to see its light sensitivity position based on a light sensitivity baseline from the distribution of the global population. Furthermore, the light sensitivity category determined earlier may also be displayed to inform the user regarding the related issues and providing some recommendations.

As shown on figures 4 to 6, the results are reported to the user in the three light conditions: the warm light, the cold light and the blinking light. A first 50 and a second 52 quantities respectively representative of a just perceptible and a very disturbing discomfort are displayed on each curve of figures 4 to 6.

According to an embodiment, the light sensitivity determining step 100 may comprise a mock step before the first measurement step wherein continuous warm light is emitted toward the user. Alternatively, any light allowing to acclimate the user's with sensation and/or symptoms may be used to have a better reliability. This mock step allows the user to better understand how the device 10 works and acclimate the user's eyes to the light emission of the device 10.

The method also comprises a resistance level determining step 150 using the method detailed above for determining a resistance level. Said resistance level is then preferably displayed on a displaying device. This resistance level determining step 150 may be performed in parallel to the light sensitivity determining step 100. Particularly, said resistance level determining step 150 is preferably performed before the results of the light sensitivity determining step 100 are displayed.

The user index determining step 200 comprises the determination of a user index representative of the level of protection required by the user, for each light environment among a group of light environments.

By "level of protection required by the user", we mean a level of protection based on answers or inputs coming from the user himself, via a questionnaire. Hence, the purpose of this user index determining step 200 is to determine the user's need protection (anamnesis), which kind of light condition does the user face and for which he needs protection. This index determining step 200 thus allows to determine, and potentially select, the light conditions from which the best filter can be chosen.

The light environments are frequent daily situations that can be a source of discomfort for the user. The group of light environments is selected among a set of environments wherein each environment of the set is associated with a given light level with different light components or characteristic (e.g. intensity, spectral, temporal and spatial components). Particularly, the environments of the set are preferably associated with different combinations of light components or characteristic from each other.

According to a preferred embodiment, each light environment is selected to depict a specific combination of the 4D light components (intensity component, spatial component, spectral component and temporal component). Hence, the group as a whole is determined to have the most representative components of the 4D light components gathered in different light environments. We mean by "*light environment*" a situation which symbolized by means of a picture and/or words a specific light configuration. For instance, a night situation may imply medium to high light intensity (intensity component) with movable light sources (spatial component) which may be only emitted toward the user during a few seconds (temporal component). The group of light environments preferably comprises at least one outdoor situation, at least one indoor situation and at least one night situation. Each light environment may be symbolized either by an image or by a description representative of the situation.

According to a preferred embodiment, the group of light environments comprises five light environments 40 which are shown in figure 7. Each light environment 40 is defined by a level in each of the 4D light components. The higher the level is, the more significant the component is. When the component has no evaluation in a component, this component is meaningless in the light environment.

As shown on figure 7, an image and/or a short description representative of each light environment 40 of the group of environments is provided to the user. In both cases, the image and the description are configured to make the user understand or imagine the light conditions induced by the described light environment 40. The description may be provided to the user either by displaying a text on a displaying device (e.g. a title) and/or via a soundtrack. For each light environment 40, the user is asked to assess the level of discomfort associated to the light environment 40 and/or the recurrence of this light environment 40. By "*recurrence*", we mean the frequency at which the user expects to face such a light environment 40.

In a preferred embodiment, questions asked to the user with regard to these light environments 40 are different depending on its light sensitivity. In other words, the user is asked to answer to a first questionnaire if he is considered as a sensitive or highly sensitive user and to a second questionnaire if he is considered as a slightly sensitive or non-sensitive user.

In this preferred embodiment, a slightly sensitive or non-sensitive determining step is performed before the user index determining step 200. Said quantity of light sensitivity threshold of the user is compared to a sensitivity reference to determine if the user is slightly sensitive or non-sensitive.

If the user is determined as sensitive or highly sensitive the first questionnaire may consist in displaying the light environments 40 and ask the user to select the light environments 4 he experiments in his daily life, as shown on figure 7.

If the user is determined as a slightly sensitive or non-sensitive user, the user index determining step 200 is performed using said second questionnaire. This second questionnaire may be specifically adapted to a slightly sensitive or non-sensitive profile. Figure 8 shows an example of said second questionnaire wherein the user is asked for each light environment 40 if he needs to close his eyes, if he blinks and wants to look away and if he doesn't have a problem.

An index 60 representative of the level of protection required by the user is determined based on either the first or the second questionnaire.

Preferably, the user interacts with the computer system to provide his answers. The answers of the user are preferably recorded on a memory of the computer system. The images of the light environments 40 are preferably displayed on the displaying device of the computer system.

A discomfort level is then be determined for each light environment 40 of the group depending on the answers of the user.

Then, a score for each light environment 40 and for a plurality of filters is determined at the score determining step 300. As shown on figure 9, the score determining step 300 comprises a step 310 for determining an index representative of the protection provided by a given filter, called the filter index. Each filter of the plurality of filter is at least defined by a filtering ability. According to a preferred embodiment, the filtering ability comprises a luminous transmittance value (*Tv*) of the given filter. The luminous transmittance (*Tv*) is a ratio of the luminous flux transmitted by the lens or filter to the incident luminous flux. The luminous transmittance defines the percentage of light from a light flux transmitted through the filter. Hence, a surface with a luminous transmittance of 0 % prevents the whole light flux to pass through the surface whereas a surface with a luminous transmittance of 100 % allows the whole light flux to pass through it without absorbing it.

The luminous transmittance in the visible spectrum may be determined using the equation as follows:

$$Tv = 100 \times \frac{\int_{380\,nm}^{780\,nm} T(\lambda) \cdot V(\lambda) \cdot S_{D65\lambda}(\lambda) \cdot d\lambda}{\int_{380\,nm}^{780\,nm} V(\lambda) \cdot S_{D65\lambda}(\lambda) \cdot d\lambda} \%$$

where

$T(\lambda)$ is the spectral transmittance of the tinted spectacle lens;
$V(\lambda)$ is the spectral luminous efficiency function for daylight (see ISO/CIE 10527);
$S_{D65\lambda}(\lambda)$ is the spectral distribution of radiation of the illuminant D 65 according to the standard of the International Commission on illumination (see ISO/CIE 10526).

The filter index determining step 310 first comprises a step of determining the filtering ability of each filter of the plurality of filter. Then, the filter index of each filter is determined using the equation as follows:

$$Filter\ index = \frac{100}{Tv}$$

The filter index thus defines the amount of light cut by the filter. A filter may have a single luminous transmittance value, i.e. a fixed value, or a plurality of luminous transmittance values, as a photochromic or electrochromic lens. In the case where the filter is a varying filter, the lower and higher luminous transmittance values are preferably calculated to determine the compliance of the filter to the user for each lower and higher luminous transmittance values.

The score determining step 300 further comprises a score calculation step 320 for determining the score for each light environment 40 among the group of light environments 40 and for each filter among a group of filters. Said score is representative of the effectiveness of a filter in a given situation, i.e. the ability of a given filter to reach the level of protection required by the user. The score for a given filter and a given light environment 40 is calculated based on the user index in said given light environment 40 and the filter index of said given filter. Particularly, the score is calculated as the ratio between the user index and the filter for said given filter and said given light environment 40. For instance, the score is equal to 1 for a filter index of 6.67 and a user index of 6.67. A score of 1 means that the filter covers 100% of the user's protection needs for said light environment 40. If a filter index is higher than 1, it means that the filter fully protects the user so that light comfort is optimal but there is a risk of vision loss.

The score is therefore a score representative of the compliance for a user of a given filter in a given light environment 40. Providing the score for selected light environment 40 which have been identified by the user as having a high level of discomfort and/or recurrence allows to help determining the best protection for the user.

Then, the filter determining step 400 comprises the determination of at least one filter based on the scores determined at the score determining step 300. This filter determining step 400 aims at ranking the filters based on their scores for the light environments 40. Preferably, the filters are ranked only with regard to the light environments selected by the user.

Each score is associated with a value representative of the compliance of a given filter in a given light environment 40 in view of the level of protection required by the user. Then, a global value may be determined based on all the values determined for a same filter. All the values of a same filter for each light environment 40 or each selected light environment 40 may be added to obtain this global value. The plurality of filters are then ranked based on the global values.

According to a preferred embodiment, one or more filters are determined for at least two transparent supports having different purposes. For example, when the transparent support is an ophthalmic lens, one or more filters are determined for at least two spectacles having different use. One spectacle may be used as sunglasses and another spectacle may be used as everyday eyeglasses. The ranking of the filters is performed with regard to specific light environment which are associated to the use of the transparent support.

According to a preferred embodiment, the closer to 100% the score is, the higher the value is. Particularly, scores from a low compliance threshold to a high compliance threshold may be associated to a positive value whereas scores out of this compliance range may be associated to a negative value. In doing so, the global value of each filter is weighted based on a predetermined degree of compliance to the user's need. For instance, the compliance range may be set from 86% to 200%. Furthermore, scores 50 which are considered to be significantly non-compliant to the user's need, e.g. scores under 50% and above 300%, may be associated to a low value. As an example, scores between 86% and 200% are associated with a value of 2, scores lower than 50% or high than 300% are associated with a value of -2 and scores from 51% to 85% or from 201% to 299% are associated with a value of -0,5. Therefore, if we consider a filter having scores equal to 42, 104, 174, 123 and 185, the respective associated values would be -2, 2, 2, 2 and 2. The global value, i.e. the sum of the values, would be 6. The scores which are summed are at least those which have been selected in the index determining step 200.

One or more filters may be then determined to have the best compliance with the user's protection need. Preferably, at least two filters from different categories of filters are determined to provide the user or the ECP with a broader list of compliant filters. Preferably, these different filter categories correspond to different purposes or for different pair of spectacles. Different categories of filter may be filters intended to be put on sunglasses and filters intended to be put on everyday eyeglasses.

As shown on figure 10, an overview of the results of the previous steps may be displayed to the user. This overview may comprise displaying the resistance level group of the user for each light condition. A gaussian diagram 42 may also be displayed showing the resistance level of the user depending on a population baseline. Light protection needs 44 for each light environment 40 and recommended products 46 are also displayed..

As shown on figure 11, one or more ophthalmic products which have been determined as the most compliant with the user's protection needs may be displayed on the displaying device of the computer system. By "ophthalmic product" we mean a filter or a transparent support as described above. Particularly, an information representative of the compliance of each ophthalmic product with regard to each light environment 40 or selected light environment 40 may be displayed. A score of each ophthalmic product may be also determined and optionally displayed for all the selected light environments 40 to give a global performance of each filter. As mentioned above, the filter determining method may be a computer-implemented method which can be performed using code instructions from a computer program product or a computer system. The computer system comprises a processor; and a memory with computer code instructions stored thereon. The memory operatively is coupled to the processor such that, when executed by the processor, the computer code instructions cause the computer system to perform the filter determining method.

In a preferred embodiment, when a resistance level is determined, said population baseline is modified to incorporate said at least two quantities representative of a light sensitivity threshold of the user to obtain a modified population baseline. Then, a resistance level is determined for a next user using said modified population baseline. In doing so, the population baseline is currently updated to make it more accurate. This update may be only partial, i.e. only a portion of the determined quantities are used to determine the modified population baseline.

This update may be performed for a predetermined group of users or users having a same characteristic. This characteristic may be a physical characteristic of the user, e.g. the age or the gender of the user, or a characteristic related to the life of the user, e.g. a geographic area. In this last example, the resistance level may therefore be determined depending on a population baseline corresponding to the country where the user lives.

This update of the population baseline may be described as follows. A first resistance level is determined based on at least two quantities representative of a light sensitivity threshold of a first user using a percentile scale based on a first population baseline. Then, an updated population baseline is determined based on said at least two quantities representative of a light sensitivity threshold of a first user and/or said first resistance level. Finally, a second resistance level is determined based on at least two quantities representative of a light sensitivity threshold of a second user using a percentile scale based on said updated population baseline. This update may be iterated a plurality of times to improve the accuracy of the population baseline. As indicated above, this update may be only partial, i.e. a user selection is made with regard to the user onto which the method is performed depending on a predetermined selection parameter.

Said update of the population baseline may be performed using artificial intelligence. In this embodiment, said method may comprise the use of a machine learning algorithm to update or adapt the population baseline with the results obtained when performing the resistance level determining method.

In a preferred embodiment, said device 10 configured to perform said resistance level determining method is a machine learning-based equipment for determining a resistance level.

The present invention further provides a method for evaluating at least one light protection level of at least one optical product intended to face an eye of a user.

An optical product according to the disclosure comprises at least one ophthalmic lens or optical filter or optical glass or optical material suitable for human vision, e.g. at least one ophthalmic lens, or optical filter, or optical film each comprising a substrate, or patch intended to be fixed on a substrate, or optical glass, or optical material intended for use in an ophthalmic instrument, for example for determining the visual acuity and/or the refraction of a subject, or any kind of safety device including a safety glass or safety wall intended to face an individual's eye, such as a protective device, for instance safety lenses or a mask or shield.

The optical product may be implemented as eyewear equipment having a frame that surrounds at least partially one or more ophthalmic lenses. By way of non-limiting example, the optical product may be a pair of glasses, sunglasses, safety goggles, sports goggles, a contact lens, an intraocular implant, an active lens with an amplitude modulation such as a polarized lens, or with a phase modulation such as an auto-focus lens, etc.

Herein, the term "lens" means an organic or inorganic glass lens, comprising a lens substrate, which may be coated with one or more coatings of various natures.

The term "ophthalmic lens" is used to mean a lens adapted to a spectacle frame, for example to protect the eye and/or correct the sight. Said lens can be chosen from afocal, unifocal, bifocal, trifocal and progressive lenses. Although ophthalmic optics is a preferred field of the invention, it will be understood that this invention can be applied to optical products of other types, such as, for example, lenses for optical instruments, in photography or astronomy, optical sighting lenses, ocular visors, optics of lighting systems, safety lenses, etc.

The at least one ophthalmic lens or optical glass or optical material suitable for human vision can provide an optical function to the user i.e. the wearer of the lens.

It can for instance be a corrective lens, namely, a power lens of the spherical, cylindrical and/or addition type for an ametropic user, for treating myopia, hypermetropia, astigmatism and/or presbyopia. The lens can have a constant power, so that it provides power as a single vision lens would do, or it can be a progressive lens having variable power.

As used herein, a base material or substrate has at least one face, i.e. a surface on one side, coated with an interferential multilayer coating providing asymmetric mirror properties.

Said base material can constitute the substrate of a lens, filter, glass, ophthalmic material etc as cited above, or can constitute the main part of a patch intended to be fixed on such substrate to provide it with asymmetric mirror properties.

The base material or substrate may also have an opposite face coated, i.e. a surface on the other side, also coated, so that, in such a case, two faces of the optical product opposite to one another may be coated.

When the base material constitutes a substrate of an ophthalmic lens, its front face is preferably coated with an antireflective or mirror coating, and its rear face preferably coated with an antireflective coating.

When the base material constitutes the main part of a patch intended to be fixed on a substrate of an ophthalmic lens, its front face is preferably coated with an antireflective or mirror coating, and its rear face will be prepared so as to be fixed on said substrate, that will be provided with an antireflective coating on its rear face.

As used herein, the rear face of the base material or the substrate is intended to mean the face which, when using the product, is the nearest from the wearer's eye, in the cases of ophthalmic lenses. It is generally a concave face. On the contrary, the front face of the substrate is the face which, when using the product, is the most distant from the wearer's eye. It is generally a convex face. The optical product can also be a planar product.

The substrate may be made of mineral glass or organic glass, preferably organic glass. The organic glasses can be either thermoplastic materials such as polycarbonates and thermoplastic polyurethanes or thermosetting (crosslinked) materials such as diethylene glycol bis(allylcarbonate) polymers and copolymers (in particular CR-39® from PPG Industries), thermosetting polyurethanes, polythiourethanes, preferably polythiourethane resins having a refractive index of 1.60 or 1.67, polyepoxides, polyepisulfides, such as those having a refractive index of 1.74, poly(meth)acrylates and copolymers based substrates, such as substrates comprising (meth)acrylic polymers and copolymers derived from bisphenol-A, polythio(meth)acrylates, as well as copolymers thereof and blends thereof. Preferred materials for the lens substrate are polycarbonates (PC), diethylene glycol bis(allylcarbonate) polymers and substrates obtained from thermosetting polythiourethane resins, which are marketed by the Mitsui Toatsu Chemicals company as MR series, in particular MR6®, MR7® and MR8® resins. The latter substrates as well as the monomers used for their preparation are especially described in the patents US 4,689,387, US 4,775,733, US 5,059,673, US 5,087,758 and US 5,191,055.

An interferential coating can be associated either by coating or by lamination, to a clear base material/substrate, i.e., a base material/substrate having a visible light mean transmission factor Tv higher than 96%, or any other base material/substrate (including non clear ones), and define with the latter an optical article having a visible light mean transmission factor Tv preferably ranging from 96 % to 4 %, more preferably from 90% to 4%. In most cases, the resulting optical article is a colored optical article.

The Tv factor, also called relative light transmission factor in the visible spectrum, relative visible light mean transmission factor or "luminous transmission" of the system, is such as defined in the standard NF EN 1836 and relates to an average in the 380-780 nm wavelength range that is weighted according to the sensitivity of the human eye at each wavelength of the range and measured under D65 illumination conditions (daylight).

As such, the interferential coating according to the invention can be tailored so as to define, with the associated substrate, different tints of sunglasses with different visible light mean transmission factors Tv:

- above 80 %,
- from 43 to 80 % (known as sunglasses of category or class 1),
- from 18 to 43 % (known as sunglasses of class 2),
- from 8 to 18% (known as sunglasses of class 3),
- below 8 % (known as sunglasses of class 4).

Said optical product may comprise a photochromic lens, an electrochromic lens, a clear lens, a blue cut function lens or a sun lens.

By "electrochromic lens", we mean a lens comprising electroactive substances which are simultaneously oxidized and reduced in contact with feed electrodes. At least some of these electroactive substances have colors that are different between their oxidized and reduced forms. The system thus changes color and / or presents a variable light absorption when an electric control that is applied between the feed electrodes is itself varied.

By "photochromic lens", we mean an ophthalmic article defined by, but not exclusive of corrective lenses, non-corrective lenses, contact lenses, intraocular lenses, magnifying lenses, protective lenses, and visors containing photochromic compounds within a coating, the lens material, a film, or any adjacent layer. Photochromic compounds undergo a transformation from one state (or form) to another state in response to certain wavelengths of electromagnetic radiation (i.e., "actinic radiation"). Each state has a characteristic absorption spectrum. For example, many photochromic compounds transform from an unactivated (e.g., bleached or substantially colorless) state to an activated (e.g., tinted) state upon exposure to actinic radiation. When the actinic radiation is removed, the photochromic compounds reversibly transform from the activated state back to the unactivated state.

Non-limiting examples of suitable organic photochromic compounds can include benzopyrans, naphthopyrans (for example naphtho[1,2-b]pyrans and naphtho[2,1-b]pyrans) spiro-9-fluoreno[1,2-b]pyrans, phenanthropyrans, quinopyrans, and indeno-fused naphthopyrans, such as those disclosed in U.S. Patent No. 5,645,767 at column 1, line 10 to column 12, line 57 and in U.S. Patent No. 5,658,501 at column 1, line 64 to column 13, line 36. Additional non-limiting examples of organic photochromic compounds that may be used include oxazines, such as benzoxazines, naphthoxazines, and spirooxazines. Other non-limiting examples of photochromic compounds that may be used include: fulgides and fulgimides, for example 3-furyl and 3-thienyl fulgides and fulgimides, which are described in U.S. Patent No. 4,931,220 at column 20, line 5 through column 21, line 38; diarylethenes, which are described in U.S. Patent Application Publication No. 2003/0174560 from paragraph [0025] to [0086]; and combinations of any of the aforementioned photochromic compounds. For example, the photochromic material (a) can comprise a compound selected from the group consisting of naphthopyrans, benzopyrans, phenanthropyrans, indenonaphthopyrans, spiro(indoline)naphthoxazines, spiro(indoline) pyridobenzoxazines, spiro(benzindoline)pyridobenzoxazines, spiro(benzindoline)naphthoxazines, spiro(indoline) benzoxazines, fulgides, fulgimides, and mixtures thereof.

Short-term light protection attributes

As shown on figure 12, the present evaluating method first comprises a step of determining a short-term light protection score 70 representative of at least one short-term light protection attribute 72 of said at least one optical product.

A short-term light protection score 70 refers to the ability of an optical product to improve comfort and light resistance during a light exposure. A short-term light protection attribute 72 thus refers to a structural, physicochemical or optical characteristic or parameter of the optical product which has an impact and comfort and light resistance during this light exposure.

Short-term light protection attributes 72 may be chosen among luminous transmittance (Tv), Speed of transmission variation (clear to dark, i.e. darkening process, and dark to clear, i.e. fading process), polarizing efficiency (PE), multi-angular reflection efficiency (Rv, Rs), blue radiation filtering in the peak emission range of LED-based digital devices ("CUT_LED", between 380 and 500nm, preferably between 430 and 470nm, most preferably between 440 and 460nm).

Luminous transmittance (Tv) is the optical product transmission perceived by the observer under specified solar radiation (%). Luminous transmittance is preferably considered as a short-term attribute for natural light environments, as a bright light and day drive environment. In this kind of light environment, the quantity of light experienced by the user is at an important level. Since luminous transmittance refers to the quantity of light which provided to the user's light through the optical product, luminous transmittance is therefore an attribute which is considered as improving short-term light protection in a bright light environment. It is defined by the mean transmittance value of a lens in the visible range 380-780 nm weighted by the irradiance solar radiation (D65) and the photopic visibility function (V lambda). The principle is to measure the spectral transmission of the optical product at reference point at normal incidence using a spectrometer. On photochromic lenses, Tv corresponds to luminous transmittance at three different states:

- Darkened/Activated state (Tv out): The photochromic lens is activated for 15 minutes in a two 150W Xenon arc lamp optical bench with a 50:50 beam splitter and the irradiance through a KG2 filter providing UVA/VIS, and the second beam having a KG2 filter and GG400 filter resulting in only supplemental VIS to provide 50 klux VIS and 6.7 $W/m^2$ UVA. A Zeiss M601 spectrophotometer then measures the Tv after 15 minutes of activation at 23°C and 35°C. Tv out is therefore the resulting average Tv of the lens in a bright light environment.
- Behind the Windshield/In car state (Tv bws): This a measurement that mimics the performance of photochromic lenses behind the windshield of a car. The photochromic lens is conditioned to achieve full unactivated state by 5 minutes activation with 365 nm ultraviolet lamp followed by heating to 70°C and exposure to yellow fluorescent lamp for 25 minutes followed by storage in the dark for at least 1 hour. Then the lens is activated using one 150W lamp on the A-BMP with a KG2 filter and a windshield filter in place and the irradiance adjusted to 1.0 $W/m^2$ integrated between 380-420 nm, 1.7 klux while at 27°C. The windshield glass consisted of a 2 plates of 2.3 mm Solar green glass with Solutia-UV enhanced polyvinylbutyrate as the laminate. The Tv bws after 15 minutes activation is then measured.
- Clear/Faded state (Tv in): The clear/faded state follows the ISO 8980-3 procedure as to what a clear/faded photochromic lens is. To measure Tv in the photochromic lens is conditioned as explained in the section concerning Tvbws above, and then Tv in is read using a Hunter UltraScan Pro, which refers to the spectral weighted transmittance associated with the vision of the eye under well-lit conditions and defined by the 1931 CIE photopic luminosity function.

Speed of transmission variation corresponds to a criterion applicable in dynamic situations only (i.e. from indoor to outdoor or opposite). Speed corresponds to activation speed for in-to-out situations and fade speed for out-to-in situations.

For an in-to-out situation, the test method consists of measuring the time of the lenses to reach 90% of the difference in transmission (Tv in - (0,9*(Tv in - Tv out))). For an in-to-out situation, this test method consists of measuring the time of the lenses to reach 70% of Tv in (0,7*Tv in). For an in-to-out situation, said test method is defined in a new standard ISO 12312- 1 (standard in progress at the time of filing).

Said polarizing efficiency (PE) is the efficiency calculation (%) according to the lens luminous transmittance measured in two cross positions under polarizing light. Principle is to measure the lens luminous transmittance (Tv with D65 source) with linear polarizing light in two positions : Lens polarizing axis (if exists) in parallel position (Tvpar) then perpendicular (Tvper) to the polarized light source. This requires implementing a linear polarizer at the output of the light source on the spectrometer. Test method is detailed in ISO 8980-3. ISO 8980-3 and ISO12312-1 specify the minimum value on polarizing efficiency to claim a lens as having polarizing function: Peff shall be not lower than 60 % for lens in category 1 and Peff shall be not lower than 78 % for lens in category 2, 3 or 4.

Said multi-angular reflection efficiency (Rv, Rs) is a criterion quantifying the overall anti-reflective efficiency whatever the light direction. Said multi-angular reflection efficiency (Rv, Rs) is preferably considered as a short-term attribute for light environments with artificial light, as night drive, indoor and screen at night environments. In this kind of light environment, a major part of light exposition is punctual light sources and peripheral light sources. Multi-angular reflection efficiency is an attribute which is considered as improving short-term light protection of the user in these light environments.

Said multi-angular reflection efficiency may be expressed as an integral of the Rv in an angular range, for example [0°- 45° ]. In a more general way, the global efficiency a of an antireflection treatment on an optical surface for angles of incidence ranging from 0° to $\theta$max can be quantified by the following parameter:
The lowest the coefficient $\alpha$, the lowest the global reflection phenomena.

$$\alpha = K \int_0^{\theta max} Rv(\theta) \sin\theta \, d\theta$$

For comparison of performances of antireflective coatings over various domains of incident angles, coefficient $\alpha$

is normalized with numeric constant K so as to have a coefficient a equal to 1% for a model Rv(θ) function being constant and equal to 1%. K depends only on θmax and is defined by the

$$\frac{1}{K} = \int_0^{\theta max} \sin\theta \, d\theta$$

following equation:

By this way, the performance of a lens over the range [0°-40°] can be compared to another lens over the range [0°-50°].

An approximate value of coefficient a may be computed by several simplifications. As Rv(θ) presents several domains for θ in which Rv is almost constant, one can define coefficient a by the following equation:

$$\frac{\alpha}{K} \approx \widehat{Rv1} \int_0^{\theta 1} \sin\theta \, d\theta + \widehat{Rv2} \int_{\theta 1}^{\theta max} \sin\theta \, d\theta$$

where Rv 1^ is the average value of Rv over the range 0° to θ1 and Rv 2 ^ is the average value of Rv over the range θ1 to θmax.

θ1 is selected so as to separate a domain of low incident angles and a domain of high incident angles including θmin. In particular, θ1 can be selected in the range of 15° to 40°.

With this definition, the two integrals of approximate coefficient a are purely geometric and can be computed exactly, and are renormalized with numeric constant K so as to have a coefficient a equal to 1% for a model Rv(θ) function being constant and equal to 1%.

In particular, θmax is higher than 35°, preferably higher than 40°. In some embodiments, θmax is higher than 45°, even better higher than 50°. The largest θmax, the more incident light is integrated in the coefficient a.

The global efficiency of an antireflection treatment on a substrate having two opposed surfaces, such as an optical lens, for angles of incidence ranging from 0° to θmax can be quantified by the following parameter:

$$\alpha 2 = K2\left(\int_0^{\theta max} Rv^{front}(\theta)\sin\theta \, d\theta + \int_0^{\theta max} Rv^{rear}(\theta)\sin\theta \, d\theta\right)$$

In which Rvfront (θ) represents the mean light reflection factor for the front main face of the substrate and Rvrear (θ) represents the mean light reflection factor for the rear main face of the substrate for an angle of incidence θ.

Approximate values for coefficient a2 may be computed in a similar manner as for coefficient a. In particular, numeric constant K2 is computed so as to have a coefficient a2 equal to 1% for model functions Rvfront (θ) and Rvrear (θ) being constant and equal to 1%.

In a specific embodiment, θmax is set to 45°, θ1 is set to 25°, Rv 1 ^ is set to Rv(15°) for front and rear faces, Rv 2 ^ is set to Rv(35°) for front and rear faces and the resulting formula for coefficient a2 is the following, the computing of which is detailed in the experimental part:

$$\alpha 2 = 0.159.(R_v^{front}(15°) + R_v^{rear}(15°)) + 0.341.(R_v^{front}(35°) + R_v^{rear}(35°)),$$

This parameter is particularly meaningful as it takes into account both low and high angles of incidence.

Low a or a2 values indicate a high multiangular efficiency of an antireflection treatment. The parameter a2 of the present optical lens is preferably lower than or equal to 0.7, more preferably lower than or equal to 0.6, even better lower than or equal to 0.55. Such a high level of multiangular efficiency of the antireflection property is attained by optical lenses having on both main faces an antireflection coating according to the invention.

The parameter α of an antireflection coating according to the invention is preferably lower than or equal to any one of the following values: 0.85, 0.75, 0.70, 0.60, 0.50, and 0.40. An antireflection coating on the front face of the lens preferably has an a parameter lower than or equal to 0.85, more preferably lower than or equal to 0.75, even better lower than or equal to 0.70, 0.60, or 0.50. An antireflection coating on the front face of the lens preferably has an a parameter lower than or equal to 0.70, more preferably lower than or equal to 0.60, even better lower than or equal to 0.50, and much better lower than or equal to 0.40.

The "mean light reflection factor," noted Rv, also called "luminous reflection", is such as defined in the ISO 13666:1998 standard, and measured in accordance with the ISO 8980-4 standard (for an angle of incidence lower than 17°, typically of 15°), i.e., this is the weighted spectral reflection average over the whole visible spectrum between 380 and 780 nm. It may be measured for all incidence angles q, thus defining a function Rv(q).

The mean light reflection factor Rv may be defined by the following equation:

$$R_V = \frac{\int_{380}^{780} R(\lambda) \cdot V(\lambda) \cdot D_{65}(\lambda) \cdot d\lambda}{\int_{380}^{780} V(\lambda) \cdot D_{65}(\lambda) \cdot d\lambda}$$

where R(λ) is the reflectance at a wavelength λ, V(λ) is the eye sensitivity function in the color space defined by the CIE (Commission on Illumination, in French "Commission Internationale de l'Eclairage") in 1931 and D65(λ) is the daylight illuminant defined in the CIE S005/E-1998 standard.

The illuminant can be adapted according to the light situation. For instance, a derived Rv function may be defined for indoor light situations using LED illuminant rather than the usual D65.

The mean light reflection factor Rv of a highly reflective coating or "mirror" coating is higher than 2.5%. The mean light reflection factor Rv of an anti-reflection coating according to the invention is preferably lower than or equal to 2.5%, more preferably lower than or equal to 2% or 1%, even more preferably ≤ 0.85 %.

Characterizing the blue-violet radiation filtering performance of a lens, associated with long-term protection, is done by calculating a weighted average cut over the blue-violet radiation from 400 to 455 nm, which corresponds to the harmful part of blue radiation as defined in ISO TR20772:2018 and in several peer-reviewed papers (Marie et al., Cell Death and Disease, 2020), (Marie et al., Cell Death and Disease, 2018), (Arnault, Barrau et al., 2013): :

$$\mathrm{BVC(B')} = 100\% - \frac{\int_{400\,nm}^{455\,nm} \mathrm{B'(\lambda) \cdot T(\lambda) \cdot d\lambda}}{\int_{400\,nm}^{455\,nm} \mathrm{B'(\lambda) \cdot d\lambda}}$$

or

$$\mathrm{BVC(B)} = 100\% - \frac{\int_{400\,nm}^{455\,nm} \mathrm{B(\lambda) \cdot T(\lambda) \cdot d\lambda}}{\int_{400\,nm}^{455\,nm} \mathrm{B(\lambda) \cdot d\lambda}}$$

**where**

T(λ) : Transmittance (%)

B'(λ) : Refined blue radiation hazard function defined in the peer-reviewed paper (Arnault et al., PlosOne, 2013) and consistent with the ISO definition of harmful blue radiation from 400 to 455 nm (ISO TR 20772:2018).

B(λ) : Blue radiation hazard function (ISO 8980-3: Annex B), much broader than the refined B', proposed in the 1970s by ICNIRP.

The way we calculate BVC and Tv are similar: for both, we weight the spectral transmittance of the lens by a biological function. For BVC, this is the blue hazard function for the retina B' and for TV, this is the photopic visual sensitivity function of the eye (V).

Beyond its impact on cumulative damage to the retina, blue radiation favors discomfort glare (Bullough, 2009) and may promote symptoms of visual fatigue, as it scatters more in the eye. In that sense, for short-term light protection, comfort is notably calculated by weighting the spectral transmittance of the lens over the whole blue range from 380 to 500nm with the spectral emission of a reference LED spectrum (CIE 015:2018) (Alexander Kokka et al 2018 Metrologia 55 526) representative of LED-based digital screens emission. The spectral range can be limited to 430- 470nm in some embodiments, or even from 440 to 460 nm, as the peak emission is around 450 nm.

Filtering in that range may also provide increased visual performance due to increased contrast sensitivity.

In order to quantify the cutting of short wavelengths i.e. blue light coming for example from car LED headlights, a parameter called the light cut factor CutLED can be used. CutLED is defined as follows:

$$Cut\ LED\ =\ 100\ -\ \frac{\int^{500}}{\int^{500}\quad LED\ emission\ (\lambda)}$$

where Σ is a discrete or continuous i.e. integral sum operator, λ is the wavelength in nm, lens T% is the spectral transmittance of the lens in % and LED emission is the spectral distribution of a white light emitting diode. As the light cut factor is a weighted function of the light source, the exact type of light source is not relevant, as soon as the main emission peak of the light source is located in a range of wavelengths between 430 nm and 480 nm, in particular between 440 nm and 465 nm.

The calculated CutLED is also indicative of the capability of filtering the solar light as the solar spectrum also comprises a high level of emission in the 430 nm - 480 nm range.

In an embodiment, LED emission is defined in% in the Cut LED formula above.

Said short-term light protection score 70 may be determined with only one short-term attribute 72 or with a combination of a plurality of short-term attributes 72.

To determine the short-term light protection score 70 from the short-term light protection attributes 72, a discrete score is determined, for instance from 0 to 4, depending on the technical values and relative to normative recommendations or standards or tests on the field. For the luminous transmittance, a continuous score may be used to be more precise in the protection level brought by the optical product.

For a bright light environment, the luminous transmittance (Tv) ranges are defined according to the above-presented standards. The higher score being assigned to the lower range Tv values. Concerning the polarization efficiency, a first threshold value is given at 78% (ISO standards). The second one at 95% is defined from our internal knowledge enabling us to discriminate product performance. Two threshold values (1 and 0.5%) for the haze are also based on internal R&D studies. The 1% value corresponds to a discomfort felt by the wearer and the 0.5% to an aesthetic threshold. In the same way for the Rv, the first threshold value at 2.5% is given by the ISO standards and the second one at 5% is defined from our internal knowledge enabling us to discriminate product performance.

For an indoor office light environment, the luminous transmittance (Tv) ranges are also defined according to the standards presented in the previous section. But the higher score is assigned to the higher range Tv values. The same features and explanation provided for a bright light environment applies here for the indoor office light with regard to α and Rv values.

Long-term light protection attributes

Said method then comprises a step of determining at least one long-term light protection score 74 representative of at least one long-term light protection attribute 76 of said at least one optical product.

A long-term light protection score refers to eye health. It thus refers to the ability of the optical product to limit the negative impact of light on eye health.

Long-term attributes may be chosen among blue radiation filtering ("CUT_LED", especially between 400 and 455nm), ultraviolet radiation protection (between 100 and 380nm) and infrared radiation protection (between 780 and 1400nm). All the short-term attributes referring to the frame may also be considered as long-term attributes because they may have an impact on eye health.

For long-term light protection, blue cut function is preferably between 400 and 455nm (see BVC(B') and BVC(B) equations above).

Said ultraviolet radiation protection corresponds to the ability of the optical product to reduce transmission from the front-side of the lens and reflection from the back-side of the lens in the UVA and UVB radiation ranges (respectively 315-380 nm and 280-315 nm), so as to allow the best health protection against UV radiations.

It is advisable for a spectacle wearer to wear before each of both eyes an ophthalmic lens that strongly reduces reflection on the rear face in the UVA- and UVB-radiation ranges, which may be harmful to the anterior part of the eye (cornea and crystalline lens).

Reflecting UV light is not really problematic on the front face of the lens, since the major part of the UV radiation which comes from the front of the wearer and might attain the wearer's eye (normal incidence, 0 to 15°) generally gets absorbed by the ophthalmic lens substrate. On the other hand, the UV radiation resulting from light sources located behind the wearer may reflect on the lens rear face and reach the wearer's eye if the lens is not provided with an antireflective coating which is efficient in the ultraviolet region, thus potentially affecting the wearer's health. It is admitted that the light rays that may reflect onto the lens rear face and reach the wearer's eye have a narrow incidence angle range, ranging from 30 to 45° (oblique incidence).

In this regard, the antireflection coating on the rear main face of the optical lens, and optionally the antireflection coating on its front main face, which exhibit very good antireflective performances in the visible region, are preferably at the same time capable of significantly reducing the UV radiation reflection, especially ultraviolet A- and ultraviolet B-rays, as compared to a bare substrate or to a substrate comprising a traditional antireflective coating that is only efficient in the visible region.

The mean reflection factor RUV on the rear main face between 280 nm and 380 nm, weighted by the function W(λ) defined in the ISO 13666:1998 standard, is preferably lower than 5 %, preferably lower than 4.5 %, more preferably lower than or equal to 4 %, even better lower than or equal to 3 % for an angle of incidence of 35°. In another embodiment, the mean reflection factor RUV on the rear main face between 280 nm and 380 nm, weighted by the function W(λ) defined in the ISO 13666:1998 standard, is preferably lower than 5 % for both an angle of incidence of 30° and for an angle of incidence of 45°. Said mean reflection factor RUV is defined through the following relation:

$$R_{UV} = \frac{\int_{280}^{380} W(\lambda).R(\lambda).d\lambda}{\int_{280}^{380} W(\lambda).d\lambda}$$

wherein R(λ) represents the lens spectral reflection factor at a given wavelength, and W(λ) represents a weighting function equal to the product of the solar spectrum irradiance Es(λ) and the efficiency relative spectral function S(λ). In certain embodiments, this factor may be measured at an angle of incidence that ranges from 30° to 45° on the rear face.

The spectral function W(λ), enabling to calculate the ultraviolet radiation transmission factors, is defined according to the ISO 13666:1998 Standard. It makes it possible to express the ultraviolet solar radiation distribution tempered by the relative spectral efficiency of such radiation for the wearer, since it simultaneously takes both the solar spectral energy Es(λ) into account, which does globally emit less UVB-rays as compared to UVA-rays, and the spectral efficiency S(λ), UVB-rays being more harmful than UVA-rays. The values for those three functions in the ultraviolet region are given in the table disclosed in ISO 13666:1998 Standard (which is reproduced at page 6 of the publication WO 2012/076714).

The above anti-UV performances are provided by the antireflection coating while maintaining low Rv factors for a wide range of angles of incidence.

When the optical product is spectacles, UV protection may also be defined using an ESPF index. The document EP 2 607 884 proposes to calculate said ESPF index that quantifies the overall reduction of ultraviolet radiation achieved by the spectacles in question and combines to do this a value of transmission through the spectacles and a value of reflection from the back face of the spectacles.

The ESPF index thus calculated makes an objective approach to evaluation of the protection from ultraviolet radiation provided by various spectacles possible. Indeed, when it is sought to protect the eyes of a wearer from ultraviolet radiation by means of spectacles, it is necessary to take into account not only radiation transmitted through the eyeglasses of these spectacles, but also rays reflected from the back face of these eyeglasses.

Said long-term light protection score 74 may be determined with only one long-term attribute 76 or with a combination of a plurality of long-term attributes 76.

At least one light protection level 78 of at least one optical product is then evaluated based on said at least one short-term 70 and at least one long-term 72 protection scores.

Beyond their ability to bring visual correction, optical lenses can thus be characterized by a large set of technical attributes that contribute differently to the modulation of light.

Said light protection level allows to evaluate an optical product considering the combination of short-term and long-term attributes to better reflect the ability of the optical product to provide comfort and light resistance as well as reduce negative impact of light on eye health.

Said light protection level may be used by displaying said at least one short-term 70 and said at least one long-term 74 light protection scores onto a same graphic or area. In other words, said short-term 70 and long-term 74 light protection scores may be used as components of the light protection level to help distinguishing how the light protection level is composed. For instance, said at least one short-term 70 and said at least one long-term 74 light protection scores may be displayed such that said at least one short-term light protection score 70 is positioned relative to said first dimension and said at least one long-term light protection score 74 is positioned relative to said second dimension, on a same graphic. Said first and second dimensions may be axes of a rectangular coordinate system, as shown on figure 19 wherein a light protection level of an optical product is positioned relative to x-axis coordinate (short-term) and y-axis coordinate (long-term).

Optical product quality attributes

As shown on figure 12, said method may also comprise a step of determining at least one vision experience score 82 representative of at least one optical product quality attribute 84 of said at least one optical product. At least one global short-term light protection score 86 is then determined based on said at least one short-term light protection score 70 and said at least one vision experience score 82. Said at least one light protection level or global light protection score 80 of said at least one optical product are then evaluated based on said at least one global short-term light protection score 86 and said at least one long-term light protection score 74.

A vision experience light protection score 82 refers to characteristics of the optical product which could degrade the comfort and light resistance of the user, as for example with poor quality lenses. This vision experience light protection score acts therefore as a weighting of the short-term light protection score 70. Preferably, said vision experience light protection score 82 is a weighting factor which is below 1. Said vision experience light protection score 82 is therefore only able to lower the short-term light protection score 70.

For each optical product quality attribute, a score between 0.5 and 1, more preferably 0.8 and 1, may be applied according to the technical values.

Optical product quality attributes 84 may be chosen among at least transmission of ghost image (Tgi), luminous transmittance (Tv), multi-angular efficiency criterion (Rv, Rs), ghost image transmission (Tgi), haze, color saturation (chroma) and yellowness (b*).

Luminous transmittance is preferably considered as an optical product quality attribute for light environments with artificial light, as night drive, indoor and screen at night environments. In this kind of light environment, a major part of light exposition is punctual light sources and peripheral light sources. As indicated above, luminous transmittance refers to a quantity of light experienced by the user is at an important level. Luminous transmittance is therefore an attribute which is considered as reducing vision experience of the user if this attribute is too high. That is why luminous transmittance is considered as an optical product quality attribute in this kind of light environment.

Multi-angular efficiency criterion (Rv, Rs) is preferably considered as an optical product quality attribute for natural light environments, as a bright light and day drive environment. In this kind of light environment, the quantity of light experienced by the user is at an important level.

Said transmission of ghost image (Tgi) is an appropriate colorimetric for evaluating ghost image intensity and visibility as defined in the European Patent Application n° 20306264.1. This ghost image is generated by an internal reflection inside the lens. It can be calculated using the photopic visibility function. This parameter may be based on the calculation of the ghost image spectrum and also includes the spectrum of the light source and the CIE 1964 observer which is found to be more relevant than the conventional CIE 1931 observer.

To calculate colorimetric parameters, in a first step, we use the transmittance spectrum of the ghost image. The transmittance spectrum enables to obtain descriptive information on the ghost image color. We derive therefrom a numerical parameter describing and evaluating ghost images concisely and accurately.

A numeric simulation tool has been developed on Matlab to calculate the transmittance spectrum of the ghost image. The transmittance spectrum of the ghost image can also be calculated on the Macleod software vStack function (available with Macleod Enhanced Edition).

The numeric simulation is based on the following assumptions:

- approximation of two parallel surfaces (lens correction not accounted),
- substrate absorption and thickness is taken into account,
- calculation made with an incidence angle of 15 degrees.

The incidence angle is set at 15 degrees to represent an average observation angle. However, any non-null incidence angle, preferably between 5 and 30 degrees, can be used if necessary to match more particular sets of conditions.

The color of the ghost image is calculated using the transmittance of the ghost image in the 380-780 nm spectral range.

The reference illuminant selected here is for example, the reference illuminant is a LED having a color temperature comprised between 2700 Kelvin and 6000 Kelvin, in particular a 4000K LED, or any point source such as a filament lamp, a halogen lamp, or even the sun (as disclosed therein, a point light source is a light source which appears small compared to the field of view of the observer through the lens. For example, the angular size of the source is more than 5-10 times smaller than the total field of view of the observer.

Color is calculated with the following tools: the necessary color calculation functions have been implemented on Matlab (also available as a commercial toolbox), and Macleod has a built-in color calculation function.

In the CIE XYZ color coordinates system, the RV of an anti-reflection coating corresponds to the Y tristimulus value (the value representing luminance) calculated from the reflectance of the anti-reflection with the 2 degrees observer (CIE 1931 observer). TGI is herein defined as the Y tristimulus value of the ghost image transmittance, calculated instead with the 10 degrees observer (or CIE 1964 observer). The 10 degrees observer is an updated version of the 2 degrees observer, providing corrections in the blue wavelength range, and is the CIE recommendation for color calculation. Like the RV, TGI is expressed in %. In other words, TGI corresponds to a colorimetric parameter representing the luminance of the color of the ghost image based on the human eye sensitivity from CIE 1964 photopic observer and based on the spectrum of a point source illuminant (instead of D65 standard reference).

The transmission of ghost image, or ghost image transmission coefficient, denoted TGI, for two-surfaces optical system is calculated by the following expression (I):

$$\mathbf{T_{GI}}(15°) = 100\frac{\int_{380}^{780} S(\lambda)\,\mathbf{T}(\lambda, 15°)\,\bar{y}_{10}(\lambda)\,d\lambda}{\int_{380}^{780} S(\lambda)\,\bar{y}_{10}(\lambda)\,d\lambda}$$

where S(λ) represents the spectrum of the light source depending on the wavelength λ in the visible spectral range between 380 nm and 780 nm, T(λ, 15°) the ghost image transmittance depending on the wavelength λ for an incidence angle of 15 degrees, and is the spectral light efficiency for a CIE 1964 photopic observer (also known as '10° observer').

The ghost image transmittance for a two-surface optical system is calculated using the following expression (II):

$$\mathbf{T}(\lambda, 15°) = T_{Cx}(\lambda, 15°).\,R_{BCc}(\lambda, \alpha).\,R_{BCx}(\lambda, \alpha).\,T_{Cc}(\lambda, \alpha).\,(T_{int}(\lambda, \alpha))^3$$

where TCx(λ, 15°) represents the spectral transmission of the incident light beam through the first (convex) surface for an incidence angle of 15 degrees depending on the wavelength λ, RBCc(λ, a) represents the spectral reflection of the light beam on the second (concave) surface 72 for a reflection occurring from the inside of the substrate medium for a refraction angle a depending on the wavelength λ, RBCx(λ, a) represents the spectral reflection of the light beam on the first (convex) surface for a reflection occurring from the inside of the substrate medium for the refraction angle a depending on the wavelength λ, TCc(λ, a) represents the spectral transmission of the light beam through the second (concave) surface for a transmission occurring from the substrate medium to the air for a refraction angle a depending on the wavelength λ, and Tint(λ, a) represents the spectral transmission of the light beam through the substrate supporting the first and second surfaces for the refraction angle a depending on the wavelength λ.

For the incidence angle of 15 degrees, the refraction angle a derives from the Snell-Descartes formula:

$$n_{air}\sin(15°) = n_{substrate}\sin(\alpha)$$

where nair is the refractive index of air, and nsubstrate is the refractive index of the lens substrate.

In the case of an optical system with two-surfaces, we have determined a numerical threshold for ghost image visibility of 0,007%. In other words, in the case N=2, the numerical threshold of ghost image visibility is identified as TGI = 0.007%.

Using the above formula TGI(15°), the coatings on the first and second surfaces can be optimized so as to obtain a transmission of ghost image that is below the numerical threshold value of ghost image visibility of 0.007%.

Figure 13 shows an example of vision experience light protection scores 82 or weighting factors correspondence to optical product quality attributes. For instance, a weighting factor of 0,8 may be applied to the short-term light protection score 70 when haze is above 1. In this example, said global light protection score 86 is determined as the product between said vision experience light protection scores 82 and said short-term light protection score 70.

## Global light protection score

Said evaluation of at least one protection level may also comprise a step of determining at least one global light protection score 80 of said at least one optical product. Said at least one global light protection score 80 is determined based on said at least one short-term 70 and at least one long-term 74 light protection scores. It allows the ECP or the user to have a single score to evaluate the light protection level of an optical product representative of both short and long-term light protection.

According to a preferred embodiment, said global light protection score 80 is calculated using the following equation.

GLOBAL LIGHT PROTECTION SCORE= Average ((ST light prot. score * VE light prot. score) ; LT light prot. score) With

1. ST light prot. score = (Attributes_1*Weight_1 + Attributes_2*Weight_2)/4
2. VE light prot. score = Attributes_1*Weight_1 + Attributes_2*Weight_2+Attributes_3*Weight_3
3. LT light prot. score = (Attributes_1*Weight_1 + Attributes_2*Weight_2)/4

Light environments

In a most preferred embodiment, said method comprises a step of determining at least one light environment which is considered as annoying in terms of light conditions. Said light environments may be chosen among a list of predetermined light environments or determined depending on the user habits and usage.

In a first embodiment where light environments are determined according to the user, the light components of said light environment may be modulated according to the light conditions which are most commonly experienced by the user or considered by the user as being the most annoying light conditions. Alternatively, said light environments may be determined using a measurement device positioned onto the user. Said measurement device may be worn by the user during a period of time which allows to measure different light environments encountered by the user and better define said light environment. Said measurement device may be integrated within said optical product to update said light environment over time.

In a second embodiment where light environments are chosen among a list of light environments, said at least one light environment is preferably chosen among a bright light environment, a day drive environment, a night drive environment, an indoor environment, a screen at night environment, an in-to-out transition environment and an out-to-in transition environment. These seven light environments have been identified, frequently described as annoying (Transitions, Light Experience Quiz, 22,660 respondents), representative of our daily lives with five static indoor or outdoor conditions and 2 transitions.

Said bright light environment may correspond to an outdoor situation representing a wearer walking in an open-view area, on a bright sunny day. Said light environment is characterized by high intensity levels and punctual light reflection (sea, building reflection, ...), ultraviolet radiation and blue radiation exposure. In this light environment, people find themselves needing to close their eyes or squint when exposed to harsh sunlight that is too intense compared to their retinal resistance. If the exposure is brief, an adaptation to the luminous flux occurs. Whereas if it is prolonged, there will be saturation of the retinal processes and blinding glare will be experienced.

Said day drive environment corresponds to an outdoor situation representing a wearer driving on a bright sunny day.

Said night drive environment corresponds to outdoor situation representing a wearer driving at night without road lighting. Vehicle headlights can result in discomfort and/or disabling glare at night. Headlights are a focal or point-like source of artificial light - the smaller the size, the more visual discomfort they will produce, and the closer they are the more disabling they will be. Individuals suffering from discomfort/disability glare while driving will transition from low light levels (nighttime) to a sudden peak of light intensity (headlights). The variation of comfort and vision will depend on the luminous intensity before the stimulation.

Said indoor environment may correspond to an indoor situation representing a wearer working on her/his computer in a daylit office. There is a high correlation between the luminance of a glare source (i.e. intensity) and the degree of discomfort. Discomforting glare perception also depends on the spatial situation of the observer in relation to the glare source. Furthermore, the more peripheral the position of the glare source, the lesser the perceived discomfort. There is also a rising concern that LED based light sources may cause more glare since they possess the characteristics of high surface luminance, small emitting size, and special spectral power distribution due to its peculiar emitting principle. Computer screens, LEDs, smartphones, and a whole array of connected devices have all had an impact on our everyday lives. Both exposure time and proximity to the source impact the visual discomfort (Coles-Brenann et al.,2019).

Said in-to-out and out-to-in transition environments correspond to transitions from indoor to outdoor and from outdoor to indoor, respectively. If a user goes from the outdoors on a bright sunny day into a dimly lit room, said user is hardly able to see our surroundings at first. As time goes by, however, said user gradually recovers his vision. This phenomenon is known as "dark adaptation", or the ability of the eye to become more visually sensitive after remaining in darkness for a period of time. On the other hand, light adaptation occurs when a user moves from the dark into bright light. The bright light momentarily dazzles said user and all he sees is white light because the sensitivity of the receptors is set to dim light. In both scenarios, time is needed to regain comfort and a good visual performance. This time is dependent on several factors, including the light level before the light variation and the significance of the light jump (sudden intensity variation). The retina takes longer to adapt again to low light flux, in those cases we often refer to the term "vision recovery.

Said method may then comprise a step of selecting at least one short-term light protection attribute 72 and at least one long-term light protection attribute 76 depending on said at least one light environment. In other words, short-term and long-term light protection attributes may be specifically chosen relative to the light environment which has been determined. The same applies to optical product quality attributes which may be selected depending on the light environment.

Figure 14 shows an example of a determination of the attributes depending on the seven light environments considered to be the most annoying. "ST Att. X" refer to short-term light protection attributes 72, "LT Att. X" refer to long-term light protection attributes 76 and "OPQ Att. X" refer to optical product quality attributes 84. These attributes are differently numbered for the sake of clarity but different attributes may refer to the same attribute. For instance, "ST Att. 1" and "ST Att.

3" may be the same attribute, e.g. the luminous transmittance (Tv). Attributes from different categories (short-term, long-term and vision experience) may also refer to a same attribute. As an example, "LT Att. 7" and "OPQ Att. 1" may be the same attribute, e.g. the multi-angular reflection efficiency (Rs, Rv).

Luminous transmittance (Tv) and polarizing efficiency (PE) may be chosen as short-term attributes when considering a bright light environment. Blue radiation filtering (BVC(B')) may preferably be considered as the unique long-term light protection attribute for night drive, indoor and screen at night environments, as UV are not emitted or do not reach the eye in such light situations.

According to a preferred embodiment, at least two light environments are considered to evaluate the light protection level of the optical product. Most preferably, each of said seven abovementioned light environments are considered for such an evaluation.

In this preferred embodiment, each of the light protection scores are determined for each light environment. In other words, at least one short-term 70, at least one long-term 74 and at least one global 80 light protection scores are determined for each determined light environment. A vision experience light protection score 82 is also determined for each light environment when such a score is determined. This preferred embodiment is for example illustrated on figure 74 wherein each light environment refers to specific short-term 70, long-term 74 and vision experience 82 light protection scores.

This embodiment is particularly relevant because the needs for protection are significantly different from one light environment to the other, which makes it more realistic to provide one score per light environment.

<u>Weightings</u>

In a first aspect, as shown on figure 15, each attribute among short-term 72 and long-term 76 light protection attributes and optical product quality attributes 84 may be weighted when determining one or more among short-term 70 and long-term 74 light protection scores and vision experience light protection score 82. For instance, polarizing efficiency (here ST Att. 2) and luminous transmittance (here ST Att. 1) may respectively consist in 25% and 75% of the short-term light protection score 70 when considering a day drive environment. As another example, multi-angular reflection efficiency (Rv), haze and color saturation (Chroma) may be equally considered when calculating the vision experience light protection score 82 for a bright light environment.

In a second aspect, a light protection evaluation score (not shown) may be determined based on each global light protection score determined for a determined light environment. Said light protection evaluation score may be determined using different weightings for said global light protection scores depending on the light environments which are considered. It allows to weight one or more light environments with respect to the other. It is particularly useful to provide such a weighting when the user is more exposed to certain environments than others or for a specific use.

In a third aspect, said global light protection score 80 may be determined using different weightings for said at least one global short-term 86 and said at least one long-term 74 light protection scores. This allows to focus the evaluation of the optical product on short-term or long-term protection while considering both of these protections.

Said evaluating method may further comprise a step of determining at least one light protection need of a user. Said light protection need may comprise one or more of at least one physiological and/or optometric parameter, at least one light exposure profile, at least one user's preference, at least one posture and eye/head behavior of the user and user's activities and associated visual and cognitive requirement.

Said physiological and/or optometric parameter may comprise age, light sensitivity level, ametropia (type and value), visual or non visual diseases (ARMD, Glaucoma, ..) and pupil behavior.

Said light exposure profile may comprise time exposure to the light, mapping of light environment and previous light exposure.

Said posture and eye/head behavior of the user may comprise head/eye position face to the light sources may also be considered (head down, straight ahead, etc.).

Said user's activities and associated visual and cognitive requirement may comprise posture and vision performance/comfort of the user that can vary according to the activities (computer work, driving, walking, gaming) for which the position of the user's head/eyes will determine different luminous radiation.

At least one optical product may be then determined for the user based on said at least one light protection level and said at least one light protection need of the user. In other words, at least one optical product may be identified as matching the light protection needs of the user.

When said optical product comprises a frame attached to an optical system, frame attributes related to said frame may be considered to evaluate said light protection level. Said frame attributes may be used when determining either said short-term light protection score, said long-term light protection score, said vision experience light protection score or for at least two of these. In other words, one or more of short-term light protection attributes, long-term light protection attributes and optical product quality attributes may comprise frame attributes.

Frame attribute may be wrap of the frame, size/thickness/shape of the frame, material and transparency of the frame or face coverage rate by the optical product (frame and/or lenses).

Said frame attributes correspond to the ability of the frame to influence light protection of the user. Indeed, said frame forms a structural obstacle to light exposition of the user's eye. Frame attributes may have an impact on light protection for each of short-term light protection, long-term light protection and vision experience. Indeed, when considering short-term and long-term protection, said frame allows to limit the light exposure of the wearer's eyes and therefore reduces discomfort and UV/IR radiations exposure. In view of short-term and long-term light protection, the more the frame covers the eyes, the more positive said frame is. To the contrary, when said frame has an important covering ability, vision experience of the user is reduced. That is why frame attributes may be evaluated positively for short-term and long-term light protections but negatively for vision experience light protection.

Example

As shown on figures 16 and 17, light protection level may be evaluated for a plurality of optical product to allow comparison between these optical products.

In this example, the light environment which is considered is a bright light environment with:

- Two dominant attributes selected for the SHORT TERM PROTECTION: Tv & PE, with weights: 0,75 (TV) & 0,25 (PE)
- Two dominant attributes selected for the LONG TERM PROTECTION: BVCb' & Espf, with weights: 0,50; 0,50
- Three dominant attributes selected for VISION EXPERIENCE: Rv, Haze, b*, with weights : 1/3; 1/3; 1/3

Here, 15 filters have been measured based on these selected attributes.

For each filter, each technical attribute is measured and reported in the table below (DATA). Then, attributes are scored based on the ranges defined above between 0 and 4 for ST and LT light protection and between 0.5 and 1 for vision experience.

Figure 16 shows a table gathering all the short-term 72 and long-term 76 light protection attributes as well as the optical product quality attributes 84 in the first upper portion of the table. Said table also gathers in the second lower portion of the table all the scores corresponding to the short-term 72 and long-term 76 light protection attributes as well as the optical product quality attributes 84.

Figure 17 then shows a table gathering all the short-term light protection score 70, long-term light protection score 74, the vision experience light protection score 82, the global short-term light protection score 86 and the global light protection score 80 for each evaluated optical product, for a given light environment.

Global light protection score 80 of figure 17 are then displayed in a graphic on figure 78 for each optical product.

Figure 19 shows a specific presentation of long-term light protection score 74 and global short-term light protection score 86. Each optical product is represented by a point having x-axis and y-axis coordinates. X-axis and y-axis coordinates respectively refer to the global short-term light protection score 86 and the long-term light protection score 74.

This presentation allows to consider both a global view of the light protection level of the optical products as well as each of its components, i.e. the global short-term light protection score 86 and the long-term light protection score 74. Indeed, the diagonal line between the x-axis and the y-axis represents the global light protection score 80. It is therefore possible to first consider the global light protection level (short-term and long-term) of each optical product by considering the position of each optical product with regard to this diagonal line.

In the meantime, each of the global short-term light protection score 86 and the long-term light protection score 74 can still be evaluated independently by only considering the x-axis or the y-axis. This kind of presentation is therefore very useful to optimize the information which is provide to an ECP or a user regarding the light protection level of optical products. The same kind of presentation can be used for different shapes of graphics, while keeping each of the global short-term light protection score 86 and the long-term light protection score 74 associated to different dimensions. For instance, the graphic may comprise bubbles, bar chart, etc.

The present invention also provides an optical product comprising a frame and at least one optical system attached to said frame and intended to face an eye of a user. The optical product is preferably spectacles comprising lenses. Said optical product comprises a data collecting device and a controller configured to perform the evaluating method which is described above

Said evaluating method may be a computer-implemented method which can be performed using code instructions from a computer program product or a computer system. The computer system comprises a processor; and a memory with computer code instructions stored thereon. The memory operatively is coupled to the processor such that, when executed by the processor, the computer code instructions cause the computer system to perform the light protection level evaluating method.

Said at least one short-term light protection attribute is a transmission attribute (Tv) and said at least one long-term attribute is a blue radiation filtering (BVC(B')).

The light protection categories for the transmission attribute (Tv) may be the following:

- first light protection category: at least 95%,
- second light protection category: between 91% and 95%,
- third light protection category: between 85% and 90%;

and wherein the light protection categories for the blue radiation filtering (BVC(B')) are the following:

- first light protection category: between 11 and 19%,
- second light protection category: between 20% and 34%,
- third light protection category: between 35% and 79%.

The method for determining at least one optical product intended to face an eye of a user preferably comprises three main phases: a first phase for determining at least one light protection need of the user wherein the eye resistance level of the user may be determined and a second phase for determining at least one light protection level of at least one optical product. In a third main phase, at least one optical product is determined for the user based on the at least one protection need and said at least one light protection level.

In this first main phase, the user may be assigned to a resistance level group, e.g. "highly sensitive", "Moderately sensitive" or "Slightly sensitive".

In the second main phase, at least one optical product is evaluated for at least one light environment using at least one short-term attribute or at least one long-term attribute. In a preferred embodiment, a plurality of optical products, i.e. at least two optical products, is evaluated for a plurality of light environments. This allows to characterize each of the optical products to then analysis if they can meet the user's light protection needs.

A selection may be performed to select the light environment experienced by the user. Hence, the optical products may be evaluated for only the selected light environments. Said at least one light environment may be selected depending on at least one subjective and/or objective data from the user. Said subjective and/or objective data from the user are at least one among a user's choice, habits of the user, data from a questionnaire fulfilled by the user and information deduced from at least one of the latter

When the second main phase is performed, each optical product has a short-term and a long-term protection scores for each selected light environment.

In a preferred embodiment, the optical products are then ranked for each selected environment depending on their short-term or long-term protection scores and assigned to a light protection category. Preferably, these light protection categories directly correspond to the resistance level groups. Indeed, the number of resistance level groups is preferably equal to the number of light protection categories. The same name can be assigned to resistance level groups and light protection categories. In an example, resistance level groups and light protection categories may comprise a first category "A" for highly sensitive users, a second category "B" for moderately sensitive users and a third category "C" for "slightly sensitive users. Assigning the optical products to a light protection category thus identify an optical product as eligible or suitable for the corresponding resistance group. A fortiori, an optical product which is eligible for a given resistance group may be also eligible for the lower resistance groups. For example, an optical product eligible for the third category, i.e. highly sensitive users, is also eligible for the second and first categories, i.e. for moderately and slightly sensitive users.

This ranking is performed for each selected light environment and for each short-term and long-term attributes determined. An example of this ranking may be seen in a table 1 shown below.

Therefore, this ranking and assigning steps allows to identify a light protection threshold when the light protection need is associated to the ranking of the optical products. Indeed, the light protection threshold corresponds, for a given light environment, to the optical product having the lowest eligible light protection score regarding the light protection need.

The third main phase consists in determining the optical products which are suitable to the user depending on the light protection need of the user. This determination may be based on a two-phase analysis described below.

A first analysis refers to the best compromise whatever the light situation or light environment selected. The optical product which is recommended or determined is the optical product with the higher number of A which correspond to the highest light protection category, or with one situation in light protection category B. If this first condition is not reached, the second analysis is performed.

A second analysis refers to the selection of a best outdoor optical product and a best indoor optical product. The optical product which is recommended or determined is the optical product in outdoor situations with the higher number of A or with one situation in light protection category B. The indoor optical product which is recommended or determined is the optical product in indoor situations with the higher number of A or with one situation in light protection category B.

Alternatively, the determination of the optical products in the third main phase may comprise both the first and the second analyses. In doing so, it may give the opportunity to suggest to the user more optical products that could suit his light protection need even if a lens perfectly suits the light protection need of the user. In this alternative applied to example 1, lens A would be recommended to the user under the first analysis and lens C could be recommended under the second analysis as a less effective optical product but potentially having other advantages as a lower price or a better looking. In this alternative, the lens C would be recommended to the user although the lens A perfectly fit the light protection need of the user.

**Example 1:**

After the evaluation of the light protection score for lenses A, B and C for light situations 1, 2, 3 and 4, the following results were obtained (see table 1 below).

Table 1

| | outdoor | | indoor | | Final reco (1) | Final reco (2) |
|---|---|---|---|---|---|---|
| | Situation 1 | Situation 2 | Situation 3 | Situations 4 | | |
| Lens A | A | A | A | A | x | |
| Lens B | A | A | C | C | | x |
| Lens C | C | B | A | A | | x |

In this example, lens A may be recommended as a suitable optical product for users belonging to the resistance level group A whatever the light situation because all light situations have been evaluated in the light protection category A. This recommendation of lens A could be performed for users belonging to any of the resistance level groups since lens A has been evaluated in the light protection category A for all light situations. In an example wherein lens A would be in light protection category B for all light situations, lens A would be recommended under the first analysis only for users belonging to resistance level groups B and C.

Lenses B and C are not a best optical product according to said first analysis but can be respectively recommended for outdoor and indoor situations.

## Claims

1. A computer-implemented method for determining a visual discomfort and/or a visual performance of a user, the method comprising the following steps:

    - Providing at least two quantities representative of a light sensitivity threshold of the user, said at least two quantities representative of a light sensitivity threshold of the user being determined by exposing the user to different light conditions;
    - determining a resistance level based on said at least two quantities representative of a light sensitivity threshold of the user using a percentile scale based on a population baseline, said eye resistance level being the capacity of the eye to manage the light intensity through photoreceptor and/or cortical processing.

2. The method according to claim 1, wherein the step of determining said resistance level comprises:

    - determining, for each quantity of said at least two quantities representative of a light sensitivity threshold of the user, a percentage value corresponding to the quantity representative of a light sensitivity threshold of the user in said percentile scale,
    - determining said resistance level depending on said percentage values.

3. A method for determining at least one optical product intended to face an eye of a user, the method comprising the following steps:

    - determining at least one light protection need of a user by performing the method for determining a visual discomfort and/or a visual performance of a user according to any one of the preceding claims,
    - determining at least one short-term light protection score representative of at least one short-term light protection attribute of said at least one optical product,
    - determining at least one long-term light protection score representative of at least one long-term light protection attribute of said at least one optical product,
    - evaluating at least one light protection level of at least one optical product based on said at least one short-term and at least one long-term protection scores,

determining at least one optical product for the user based on said at least one light protection level and said at least one light protection need of the user.

**4.** The method according to claim 3, wherein the light sensitivity threshold is determined using a device configured to expose the user to an increasing light level and to determine the at least one quantity representative of the user's light sensitivity threshold based on a user's feedback representative of a discomfort.

**5.** The method according to claim 3 or 4, further comprising:

- determining at least one light environment,
- selecting said at least one short-term light protection attribute and said at least one long-term light protection attribute respectively among a group of short-term light protection attributes and a group of long-term light protection attributes, depending on said at least one determined light environment.

**6.** The method according to claim 5, wherein said at least one light environment are selected within a group of light environments, said group of light environments comprises at least one among a bright light environment, a day drive environment, a night drive environment, an indoor environment, a screen at night environment, an in-to-out transition environment and an out-to-in transition environment.

**7.** The method according to any one of claims 3 to 6, wherein said at least one short-term light protection attribute is a transmission attribute (Tv) and said at least one long-term attribute is a blue radiation filtering (BVC(B')).

**8.** The method according to any one of claims 3 to 7, further comprising:

- determining at least one vision experience score representative of at least one optical product quality attribute of said at least one optical product,
- determining at least one global short-term light protection score based on said at least one short-term light protection score and said at least one vision experience score,

wherein said at least one light protection level of said at least one optical product is evaluated based on said at least one global short-term light protection score and said at least one long-term light protection score.

**9.** The method according to any one of claims 3 to 8, wherein the step of evaluating at least one protection level comprises a step of determining at least one global light protection score of said at least one optical product based on said at least one short-term and at least one long-term light protection scores.

**10.** The method according to claim 9, further comprising:

- determining at least two light environments,

wherein at least one short-term, at least one long-term and at least one global light protection scores are determined for each determined light environment.

**11.** The method according to any one of claims 3 to 10, wherein said at least one short-term light protection attribute is one among a transmission attribute, a polarization attribute, a stray light attribute and a spectral attribute.

**12.** The method according to any one of claims 3 to 11, wherein said at least one long-term attribute is a spectral attribute.

**13.** A computer system for determining a visual discomfort and/or a visual performance of a user, the system comprising:

- a processor; and
- a memory with computer code instructions stored thereon, the memory operatively coupled to the processor such that, when executed by the processor, the computer code instructions cause the computer system to perform a method for determining at least one optical product intended to face an eye of a user, the method comprising the following steps:
- determining at least one light protection need of a user by:

  • providing at least two quantities representative of a light sensitivity threshold of the user, said at least two quantities representative of a light sensitivity threshold of the user being determined by exposing the user to different light conditions;

• determining a resistance level based on said at least two quantities representative of a light sensitivity threshold of the user using a percentile scale based on a population baseline, said eye resistance level being the capacity of the eye to manage the light intensity through photoreceptor and/or cortical processing,

- determining at least one short-term light protection score representative of at least one short-term light protection attribute of said at least one optical product,
- determining at least one long-term light protection score representative of at least one long-term light protection attribute of said at least one optical product,
- evaluating at least one light protection level of at least one optical product based on said at least one short-term and at least one long-term protection scores.

**14.** An optical product comprising a frame and at least one optical system attached to said frame and intended to face an eye of a user, said optical product comprising a data collecting device and a controller configured to perform a method for determining at least one optical product intended to face an eye of a user, the method comprising the following steps:

- determining at least one light protection need of a user by:

• providing at least two quantities representative of a light sensitivity threshold of the user, said at least two quantities representative of a light sensitivity threshold of the user being determined by exposing the user to different light conditions;

• determining a resistance level based on said at least two quantities representative of a light sensitivity threshold of the user using a percentile scale based on a population baseline, said eye resistance level being the capacity of the eye to manage the light intensity through photoreceptor and/or cortical processing,

- determining at least one short-term light protection score representative of at least one short-term light protection attribute of said at least one optical product,
- determining at least one long-term light protection score representative of at least one long-term light protection attribute of said at least one optical product,
- evaluating at least one light protection level of at least one optical product based on said at least one short-term and at least one long-term protection scores,
- determining at least one optical product for the user based on said at least one light protection level and said at least one light protection need of the user.

**Patentansprüche**

**1.** Computerimplementiertes Verfahren zum Ermitteln eines visuellen Unbehagens und/oder einer visuellen Leistungsfähigkeit eines Benutzers, wobei das Verfahren die folgenden Schritte umfasst:

- Bereitstellen von mindestens zwei Größen, die einen Lichtempfindlichkeitsschwellenwert des Benutzers repräsentieren, wobei die mindestens zwei Größen, die einen Lichtempfindlichkeitsschwellenwert des Benutzers repräsentieren, ermittelt werden, indem der Benutzer unterschiedlichen Lichtbedingungen ausgesetzt wird;
- Ermitteln eines Widerstandsgrads basierend auf den mindestens zwei Größen, die einen Lichtempfindlichkeitsschwellenwert des Benutzers repräsentieren, indem eine Perzentilskala verwendet wird, die auf einer Bevölkerungsbasislinie basiert ist, wobei der Widerstandsgrad des Auges die Fähigkeit des Auges ist, durch Photorezeptoren und/oder eine Verarbeitung im Cortex mit der Lichtintensität umzugehen.

**2.** Verfahren nach Anspruch 1, wobei der Schritt des Ermittelns des Widerstandsgrads umfasst:

- Ermitteln, für jede Größe der mindestens zwei Größen, die einen Lichtempfindlichkeitsschwellenwert des Benutzers repräsentieren, eines Prozentwerts, welcher der Größe entspricht, die einen Lichtempfindlichkeitsschwellenwert des Benutzers auf der Perzentilskala entspricht,
- Ermitteln des Widerstandsgrads in Abhängigkeit von dem Prozentwert.

**3.** Verfahren zum Ermitteln von mindestens einem optischen Produkt, das dafür vorgesehen ist, einem Auge eines Benutzers zugewandt zu werden, wobei das Verfahren die folgenden Schritte umfasst:

- Ermitteln von mindestens einem Lichtschutzbedürfnis eines Benutzers, indem das Verfahren zum Ermitteln eines visuellen Unbehagens und/oder einer visuellen Leistungsfähigkeit eines Benutzers nach einem der vorhergehenden Ansprüche durchgeführt wird,
- Ermitteln von mindestens einer Kurzzeit-Lichtschutzbewertung, die mindestens ein Kurzzeit-Lichtschutzattribut des mindestens einen optischen Produkts repräsentiert,
- Ermitteln von mindestens einer Langzeit-Lichtschutzbewertung, die mindestens ein Langzeit-Lichtschutzattribut des mindestens einen optischen Produkts repräsentiert,
- Beurteilen von mindestens einem Lichtschutzgrad von mindestens einem optischen Produkt basierend auf der mindestens einen Kurzzeit- und der mindestens einen Langzeit-Schutzbewertung,

Ermitteln von mindestens einem optischen Produkt für den Benutzer basierend auf dem mindestens einen Lichtschutzgrad und dem mindestens einen Lichtschutzbedürfnis des Benutzers.

**4.** Verfahren nach Anspruch 3, wobei der Lichtempfindlichkeitsschwellenwert unter Verwendung einer Vorrichtung ermittelt wird, die konfiguriert ist, um den Benutzer einem zunehmenden Lichtpegel auszusetzen und um die mindestens eine Größe, die den Lichtempfindlichkeitsschwellenwert des Benutzers repräsentiert, basierend auf einer Rückmeldung des Benutzers zu ermitteln, die ein Unbehagen repräsentiert.

**5.** Verfahren nach Anspruch 3 oder 4, das ferner umfasst:

- Ermitteln mindestens einer Lichtumgebung,
- Auswählen des mindestens einen Kurzzeit-Lichtschutzattributs und des mindestens einen Langzeit-Lichtschutzattributs aus einer Gruppe von Kurzzeit-Lichtschutzattributen bzw. einer Gruppe von Langzeit-Lichtschutzattributen in Abhängigkeit von der mindestens einen ermittelten Lichtumgebung.

**6.** Verfahren nach Anspruch 5, wobei die mindestens eine Lichtumgebung innerhalb einer Gruppe von Lichtumgebungen ausgewählt wird, wobei die Gruppe von Lichtumgebungen mindestens eine von einer hellen Lichtumgebung, einer Tagesfahrtumgebung, einer Nachtfahrtumgebung, einer Innenraumumgebung, eines Bildschirms in einer Nachtumgebung, einer Übergangsumgebung von drinnen nach draußen und einer Übergangsumgebung von draußen nach drinnen umfasst.

**7.** Verfahren nach einem der Ansprüche 3 bis 6, wobei das mindestens eine Kurzzeit-Lichtschutzattribut ein Transmissionsattribut (Tv) ist und wobei das mindestens eine Langzeit-Lichtschutzattribut eine Blaustrahlungsfilterung (BVC(B')) ist.

**8.** Verfahren nach einem der Ansprüche 3 bis 7, das ferner umfasst:

- Ermitteln mindestens einer Sichterfahrungsbewertung, die mindestens ein Qualitätsattribut eines optischen Produkts des mindestens einen optischen Produkts repräsentiert,
- Ermitteln mindestens einer globalen Kurzzeit-Lichtschutzbewertung basierend auf der mindestens einen Kurzzeit-Lichtschutzbewertung und der mindestens einen Sichterfahrungsbewertung,

wobei der mindestens eine Lichtschutzgrad des mindestens einen optischen Produkts basierend auf der mindestens einen globalen Kurzzeit-Lichtschutzbewertung und der mindestens einen Langzeit-Schutzbewertung beurteilt wird.

**9.** Verfahren nach einem der Ansprüche 3 bis 8, wobei der Schritt des Beurteilens von mindestens einem Schutzgrad einen Schritt des Ermittelns von mindestens einer globalen Lichtschutzbewertung des mindestens einen optischen Produkts basierend auf der mindestens einen Kurzzeit- und der mindestens einen Langzeit-Lichtschutzbewertung umfasst.

**10.** Verfahren nach Anspruch 9, das ferner umfasst:

- Ermitteln von mindestens zwei Lichtumgebungen,

wobei für jede ermittelte Lichtumgebung mindestens eine Kurzzeit- mindestens eine Langzeit- und mindestens eine globale Lichtschutzbewertung ermittelt werden.

**11.** Verfahren nach einem der Ansprüche 3 bis 10, wobei das mindestens eine Kurzzeit-Lichtschutzattribut eines von einem Transmissionsattribut, einem Polarisationsattribut, einem Streulichtattribut und einem Spektralattribut ist.

**12.** Verfahren nach einem der Ansprüche 3 bis 11, wobei das mindestens Langzeitattribut ein Spektralattribut ist.

**13.** Computersystem zum Ermitteln eines visuellen Unbehagens und/oder einer visuellen Leistungsfähigkeit eines Benutzers, wobei das System umfasst:

- einen Prozessor; und
- einen Speicher, in dem Computercodeanweisungen gespeichert sind, wobei der Speicher funktionsfähig mit dem Prozessor gekoppelt ist, sodass die Computercodeanweisungen, wenn sie von dem Prozessor ausgeführt werden, das Computersystem veranlassen, ein Verfahren zum Ermitteln von mindestens einem optischen Produkt, das dafür vorgesehen ist, einem Auge eines Benutzers zugewandt zu werden, wobei das Verfahren die folgenden Schritte umfasst:
- Ermitteln von mindestens einem Lichtschutzbedürfnis eines Benutzers durch:

  • Bereitstellen von mindestens zwei Größen, die einen Lichtempfindlichkeitsschwellenwert des Benutzers repräsentieren, wobei die mindestens zwei Größen, die einen Lichtempfindlichkeitsschwellenwert des Benutzers repräsentieren, ermittelt werden, indem der Benutzer unterschiedlichen Lichtbedingungen ausgesetzt wird;
  • Ermitteln von einem Widerstandsgrad basierend auf den mindestens zwei Größen, die einen Lichtempfindlichkeitsschwellenwert des Benutzers repräsentieren, indem eine Perzentilskala verwendet wird, die auf einer Bevölkerungsbasislinie basiert ist, wobei der Widerstandsgrad des Auges die Fähigkeit des Auges ist, durch Photorezeptoren und/oder eine Verarbeitung im Cortex mit der Lichtintensität umzugehen.

- Ermitteln von mindestens einer Kurzzeit-Lichtschutzbewertung, die mindestens ein Kurzzeit-Lichtschutzattribut des mindestens einen optischen Produkts repräsentiert,
- Ermitteln von mindestens einer Langzeit-Lichtschutzbewertung, die mindestens ein Langzeit-Lichtschutzattribut des mindestens einen optischen Produkts repräsentiert,
- Beurteilen von mindestens einem Lichtschutzgrad von mindestens einem optischen Produkt basierend auf der mindestens einen Kurzzeit- und der mindestens einen Langzeit-Schutzbewertung.

**14.** Optisches Produkt, das einen Rahmen und mindestens ein optisches System umfasst, das an dem Rahmen befestigt ist, und das dafür vorgesehen ist, einem Auge eines Benutzers zugewandt zu werden, wobei das optische Produkt eine Datenerfassungsvorrichtung und eine Steuereinheit umfasst, die konfiguriert ist zum Durchführen eines Verfahrens zum Ermitteln von mindestens einem optischen Produkt, das dafür vorgesehen ist, einem Auge eines Benutzers zugewandt zu werden, wobei das Verfahren die folgenden Schritte umfasst:

- Ermitteln von mindestens einem Lichtschutzbedürfnis eines Benutzers durch:

  • Bereitstellen von mindestens zwei Größen, die einen Lichtempfindlichkeitsschwellenwert des Benutzers repräsentieren, wobei die mindestens zwei Größen, die einen Lichtempfindlichkeitsschwellenwert des Benutzers repräsentieren, ermittelt werden, indem der Benutzer unterschiedlichen Lichtbedingungen ausgesetzt wird;
  • Ermitteln von einem Widerstandsgrad basierend auf den mindestens zwei Größen, die einen Lichtempfindlichkeitsschwellenwert des Benutzers repräsentieren, indem eine Perzentilskala verwendet wird, die auf einer Bevölkerungsbasislinie basiert ist, wobei der Widerstandsgrad des Auges die Fähigkeit des Auges ist, durch Photorezeptoren und/oder eine Verarbeitung im Cortex mit der Lichtintensität umzugehen.

- Ermitteln von mindestens einer Kurzzeit-Lichtschutzbewertung, die mindestens ein Kurzzeit-Lichtschutzattribut des mindestens einen optischen Produkts repräsentiert,
- Ermitteln von mindestens einer Langzeit-Lichtschutzbewertung, die mindestens ein Langzeit-Lichtschutzattribut des mindestens einen optischen Produkts repräsentiert,
- Beurteilen von mindestens einem Lichtschutzgrad von mindestens einem optischen Produkt basierend auf der mindestens einen Kurzzeit- und der mindestens einen Langzeit-Schutzbewertung,
- Ermitteln von mindestens einem optischen Produkt für den Benutzer basierend auf dem mindestens einen Lichtschutzgrad und dem mindestens einen Lichtschutzbedürfnis des Benutzers.

## Revendications

1. Procédé mis en œuvre par ordinateur pour déterminer une gêne visuelle et/ou une performance visuelle d'un utilisateur, le procédé comprenant les étapes suivantes :

   - obtention d'au moins deux quantités représentatives d'un seuil de sensibilité à la lumière de l'utilisateur, lesdites au moins deux quantités représentatives d'un seuil de sensibilité à la lumière de l'utilisateur étant déterminées par exposition de l'utilisateur à différentes conditions lumineuses ;
   - détermination d'un niveau de résistance sur la base desdites au moins deux quantités représentatives d'un seuil de sensibilité à la lumière de l'utilisateur au moyen d'une échelle de centiles basée sur une ligne de base de la population, ledit niveau de résistance oculaire étant la capacité de l'œil à gérer l'intensité lumineuse par le biais d'un traitement par les photorécepteurs et/ou cortical.

2. Procédé selon la revendication 1, dans lequel l'étape de détermination dudit niveau de résistance comprend :

   - la détermination, pour chaque quantité parmi lesdites au moins deux quantités représentatives d'un seuil de sensibilité à la lumière de l'utilisateur, d'une valeur de pourcentage correspondant à la quantité représentative d'un seuil de sensibilité à la lumière de l'utilisateur sur ladite échelle des centiles,
   - la détermination dudit niveau de résistance en fonction desdites valeurs de pourcentage.

3. Procédé de détermination d'au moins un produit optique destiné à faire face à un œil d'un utilisateur, le procédé comprenant les étapes suivantes :

   - détermination d'au moins un besoin de protection contre la lumière d'un utilisateur par réalisation du procédé de détermination d'une gêne visuelle et/ou d'une performance visuelle d'un utilisateur selon l'une quelconque des revendications précédentes,
   - détermination d'au moins un score de protection contre la lumière à court terme représentatif d'au moins un attribut de protection contre la lumière à court terme dudit au moins un produit optique,
   - détermination d'au moins un score de protection contre la lumière à long terme représentatif d'au moins un attribut de protection contre la lumière à long terme dudit au moins un produit optique,
   - évaluation d'au moins un niveau de protection contre la lumière d'au moins un produit optique sur la base desdits au moins un score de protection à court terme et au moins un score de protection à long terme,

   détermination d'au moins un produit optique pour l'utilisateur sur la base dudit au moins un niveau de protection contre la lumière et dudit au moins un besoin de protection contre la lumière de l'utilisateur.

4. Procédé selon la revendication 3, dans lequel le seuil de sensibilité à la lumière est déterminé au moyen d'un dispositif conçu pour exposer l'utilisateur à un niveau de lumière croissant et pour déterminer l'au moins une quantité représentative du seuil de sensibilité à la lumière de l'utilisateur sur la base d'un retour de l'utilisateur représentatif d'une gêne.

5. Procédé selon la revendication 3 ou 4, comprenant en outre :

   - la détermination d'au moins un environnement lumineux,
   - la sélection dudit au moins un attribut de protection contre la lumière à court terme et dudit au moins un attribut de protection contre la lumière à long terme respectivement dans un groupe d'attributs de protection contre la lumière à court terme et un groupe d'attributs de protection contre la lumière à long terme, en fonction dudit au moins un environnement lumineux déterminé.

6. Procédé selon la revendication 5, dans lequel ledit au moins un environnement lumineux est choisi dans un groupe d'environnements lumineux, ledit groupe d'environnements lumineux comprend au moins un environnement parmi un environnement de lumière vive, un environnement de conduite de jour, un environnement de conduite de nuit, un environnement intérieur, un environnement d'écran la nuit, un environnement de transition de l'intérieur vers l'extérieur et un environnement de transition de l'extérieur vers l'intérieur.

7. Procédé selon l'une quelconque des revendications 3 à 6, dans lequel ledit au moins un attribut de protection contre la lumière à court terme est un attribut de transmission (TV) et ledit au moins un attribut à long terme est un filtrage du rayonnement bleu (BVC(B')).

**8.** Procédé selon l'une quelconque des revendications 3 à 7, comprenant en outre :

- la détermination d'au moins un score d'expérience de vision représentatif d'au moins un attribut de qualité de produit optique dudit au moins un produit optique,
- la détermination d'au moins un score global de protection contre la lumière à court terme sur la base dudit au moins un score de protection contre la lumière à court terme et dudit au moins un score d'expérience de vision,

dans lequel ledit au moins un niveau de protection contre la lumière dudit au moins un produit optique est évalué sur la base dudit au moins un score global de protection contre la lumière à court terme et dudit au moins un score de protection contre la lumière à long terme.

**9.** Procédé selon l'une quelconque des revendications 3 à 8, dans lequel l'étape d'évaluation d'au moins un niveau de protection comprend une étape de détermination d'au moins un score global de protection contre la lumière dudit au moins un produit optique sur la base desdits au moins un score de protection contre la lumière à court terme et au moins un score de protection contre la lumière à long terme.

**10.** Procédé selon la revendication 9, comprenant en outre :

- la détermination d'au moins deux environnements lumineux,

dans lequel au moins un score de protection contre la lumière à court terme, au moins un score de protection contre la lumière à long terme et au moins un score global de protection contre la lumière sont déterminés pour chaque environnement lumineux déterminé.

**11.** Procédé selon l'une quelconque des revendications 3 à 10, dans lequel ledit au moins un attribut de protection contre la lumière à court terme est un attribut parmi un attribut de transmission, un attribut de polarisation, un attribut de lumière parasite et un attribut spectral.

**12.** Procédé selon l'une quelconque des revendications 3 à 11, dans lequel ledit au moins un attribut à long terme est un attribut spectral.

**13.** Système informatique destiné à déterminer une gêne visuelle et/ou une performance visuelle d'un utilisateur, le système comprenant :

- un processeur ; et
- une mémoire sur laquelle sont stockées des instructions de code informatique, la mémoire étant fonctionnellement couplée au processeur de telle sorte que, lors de leur exécution par le processeur, les instructions de code informatique conduisent le système informatique à réaliser un procédé de détermination d'au moins un produit optique destiné à faire face à un œil d'un utilisateur, le procédé comprenant les étapes suivantes :
- détermination d'au moins un besoin de protection contre la lumière d'un utilisateur par :

  • obtention d'au moins deux quantités représentatives d'un seuil de sensibilité à la lumière de l'utilisateur, lesdites au moins deux quantités représentatives d'un seuil de sensibilité à la lumière de l'utilisateur étant déterminées par exposition de l'utilisateur à différentes conditions lumineuses ;
  • détermination d'un niveau de résistance sur la base desdites au moins deux quantités représentatives d'un seuil de sensibilité à la lumière de l'utilisateur au moyen d'une échelle de centiles basée sur une ligne de base de la population, ledit niveau de résistance oculaire étant la capacité de l'œil à gérer l'intensité lumineuse par le biais d'un traitement par les photorécepteurs et/ou cortical,

- détermination d'au moins un score de protection contre la lumière à court terme représentatif d'au moins un attribut de protection contre la lumière à court terme dudit au moins un produit optique,
- détermination d'au moins un score de protection contre la lumière à long terme représentatif d'au moins un attribut de protection contre la lumière à long terme dudit au moins un produit optique,
- évaluation d'au moins un niveau de protection contre la lumière d'au moins un produit optique sur la base desdits au moins un score de protection à court terme et au moins un score de protection à long terme.

**14.** Produit optique comprenant une monture et au moins un système optique fixé à ladite monture et destiné à faire face à un œil d'un utilisateur, ledit produit optique comprenant un dispositif de collecte de données et un contrôleur conçu pour réaliser un procédé de détermination d'au moins un produit optique destiné à faire face à un œil d'un utilisateur, le procédé comprenant les étapes suivantes :

- détermination d'au moins un besoin de protection contre la lumière d'un utilisateur par :

  • obtention d'au moins deux quantités représentatives d'un seuil de sensibilité à la lumière de l'utilisateur, lesdites au moins deux quantités représentatives d'un seuil de sensibilité à la lumière de l'utilisateur étant déterminées par exposition de l'utilisateur à différentes conditions lumineuses ;
  • détermination d'un niveau de résistance sur la base desdites au moins deux quantités représentatives d'un seuil de sensibilité à la lumière de l'utilisateur au moyen d'une échelle de centiles basée sur une ligne de base de la population, ledit niveau de résistance oculaire étant la capacité de l'œil à gérer l'intensité lumineuse par le biais d'un traitement par les photorécepteurs et/ou cortical,

- détermination d'au moins un score de protection contre la lumière à court terme représentatif d'au moins un attribut de protection contre la lumière à court terme dudit au moins un produit optique,
- détermination d'au moins un score de protection contre la lumière à long terme représentatif d'au moins un attribut de protection contre la lumière à long terme dudit au moins un produit optique,
- évaluation d'au moins un niveau de protection contre la lumière d'au moins un produit optique sur la base desdits au moins un score de protection à court terme et au moins un score de protection à long terme,
- détermination d'au moins un produit optique pour l'utilisateur sur la base dudit au moins un niveau de protection contre la lumière et dudit au moins un besoin de protection contre la lumière de l'utilisateur.

Fig. 3

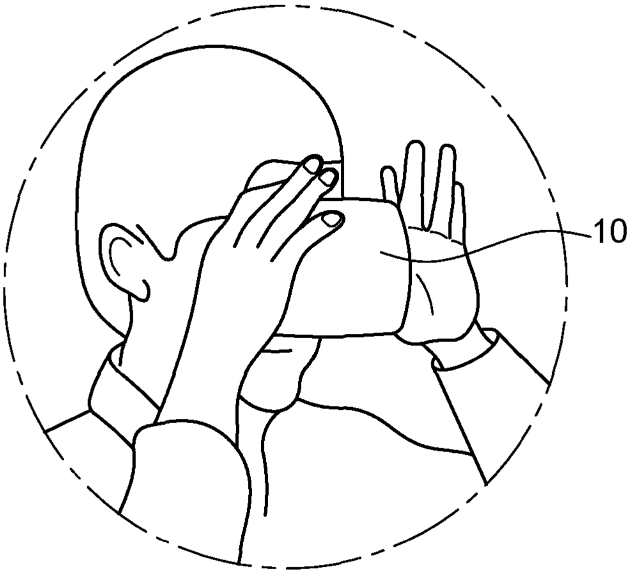


Fig. 4

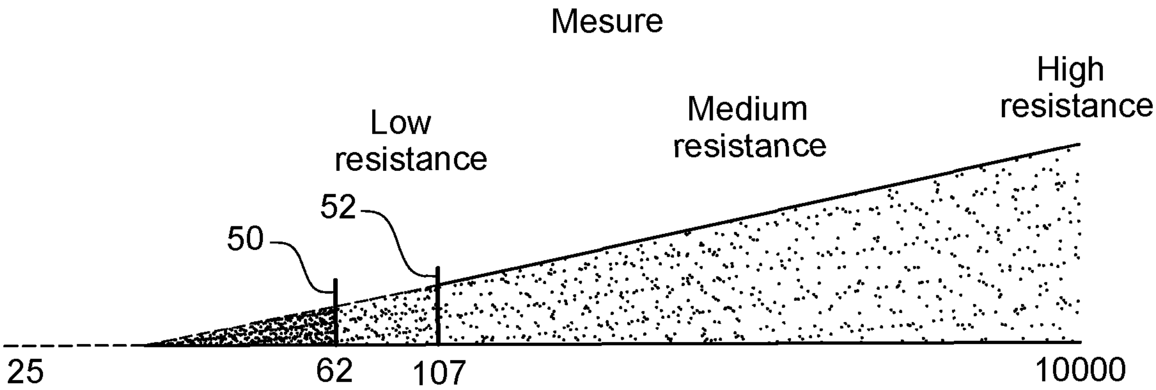


Fig. 5

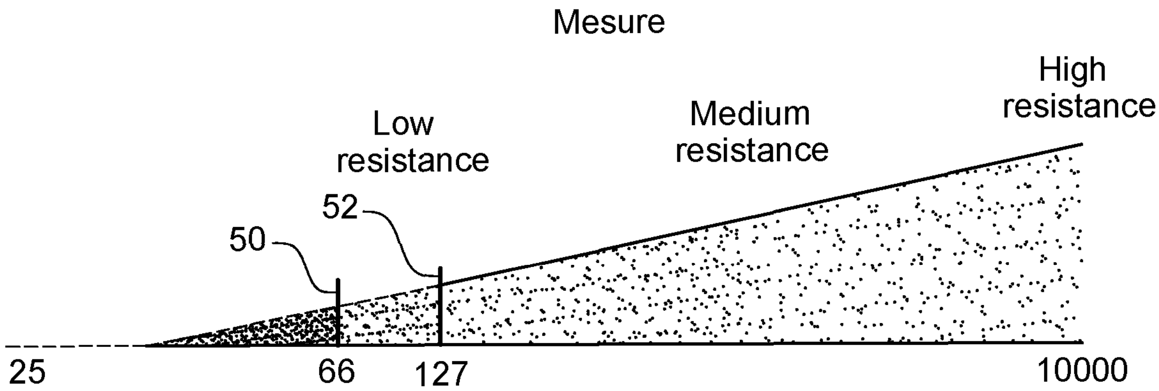

Fig. 6

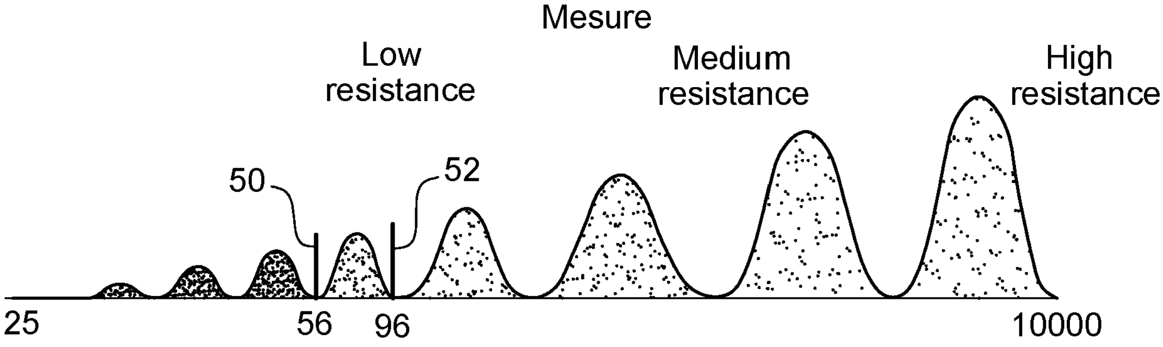


Fig. 7

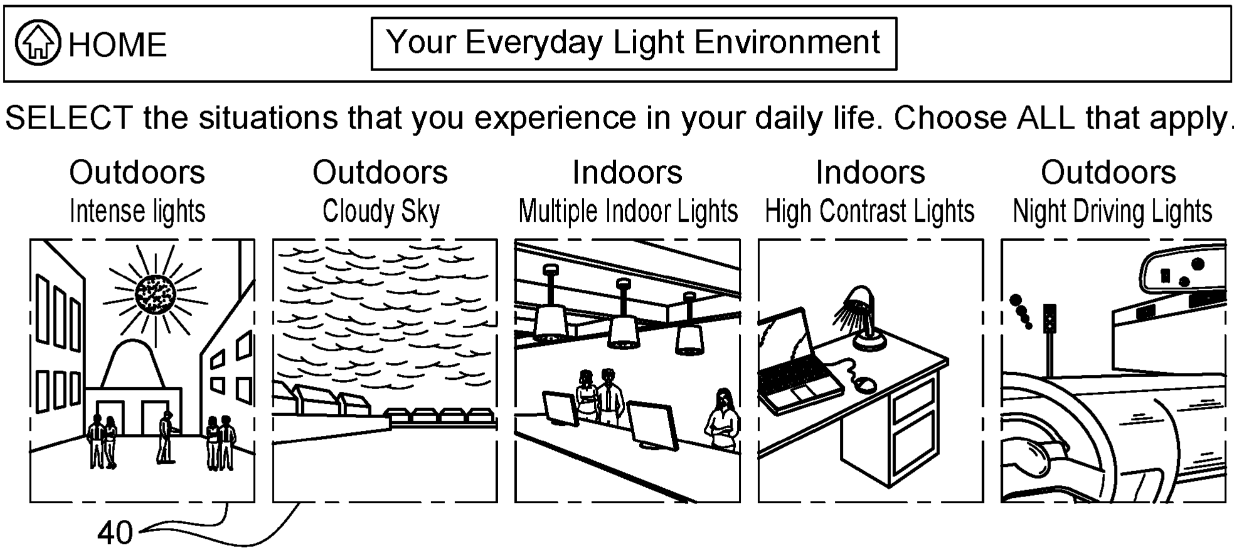


Fig. 8

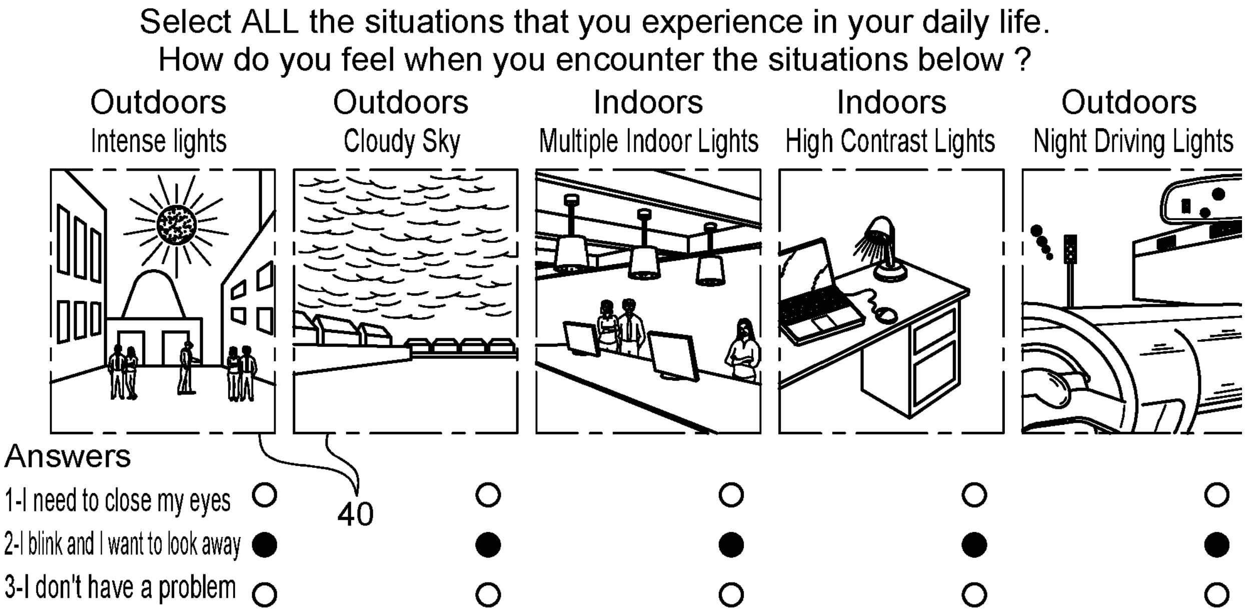

Fig. 9

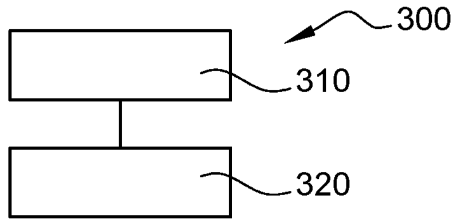
300
310
320

Fig. 10

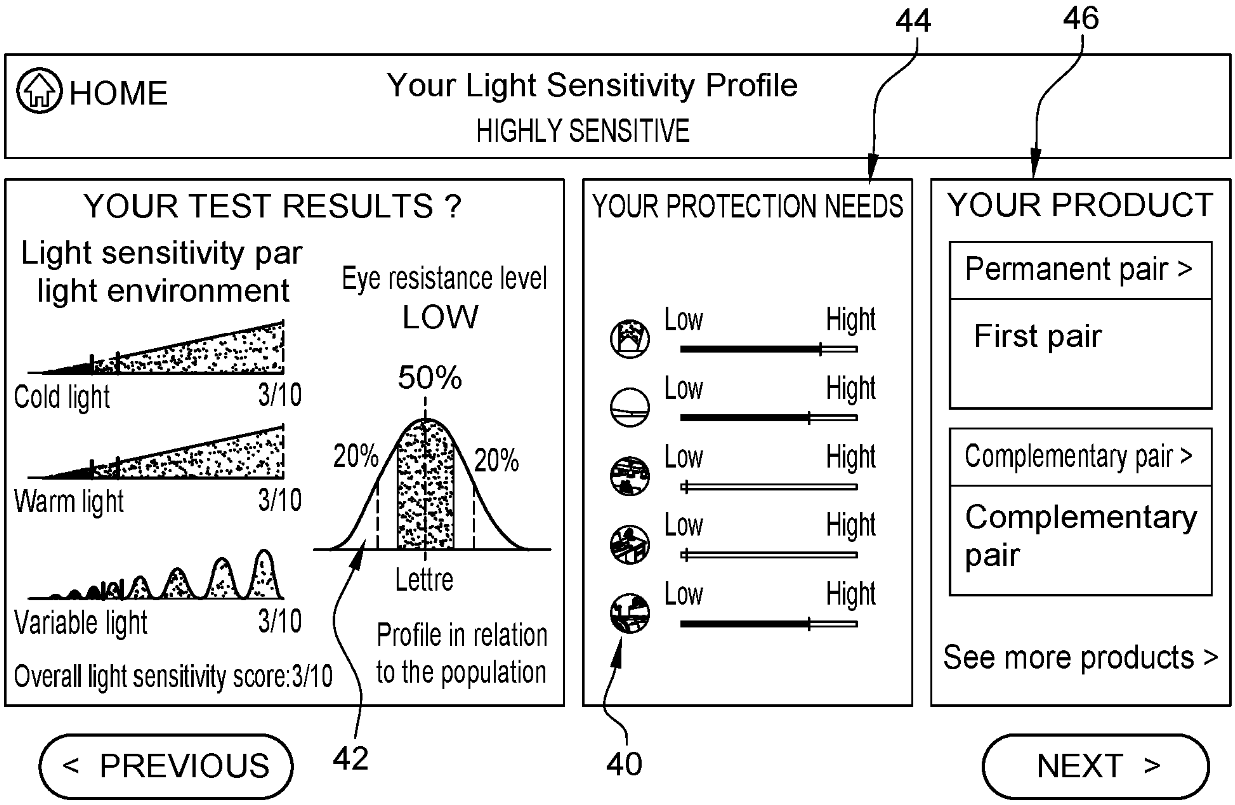
44
46
HOME
Your Light Sensitivity Profile
HIGHLY SENSITIVE
YOUR TEST RESULTS ?
Light sensitivity par light environment
Cold light 3/10
Warm light 3/10
Variable light 3/10
Overall light sensitivity score:3/10
Eye resistance level
LOW
50%
20%
20%
Lettre
Profile in relation to the population
YOUR PROTECTION NEEDS
Low Hight
Low Hight
Low Hight
Low Hight
Low Hight
YOUR PRODUCT
Permanent pair >
First pair
Complementary pair >
Complementary pair
See more products >
< PREVIOUS
42
40
NEXT >

Fig. 11

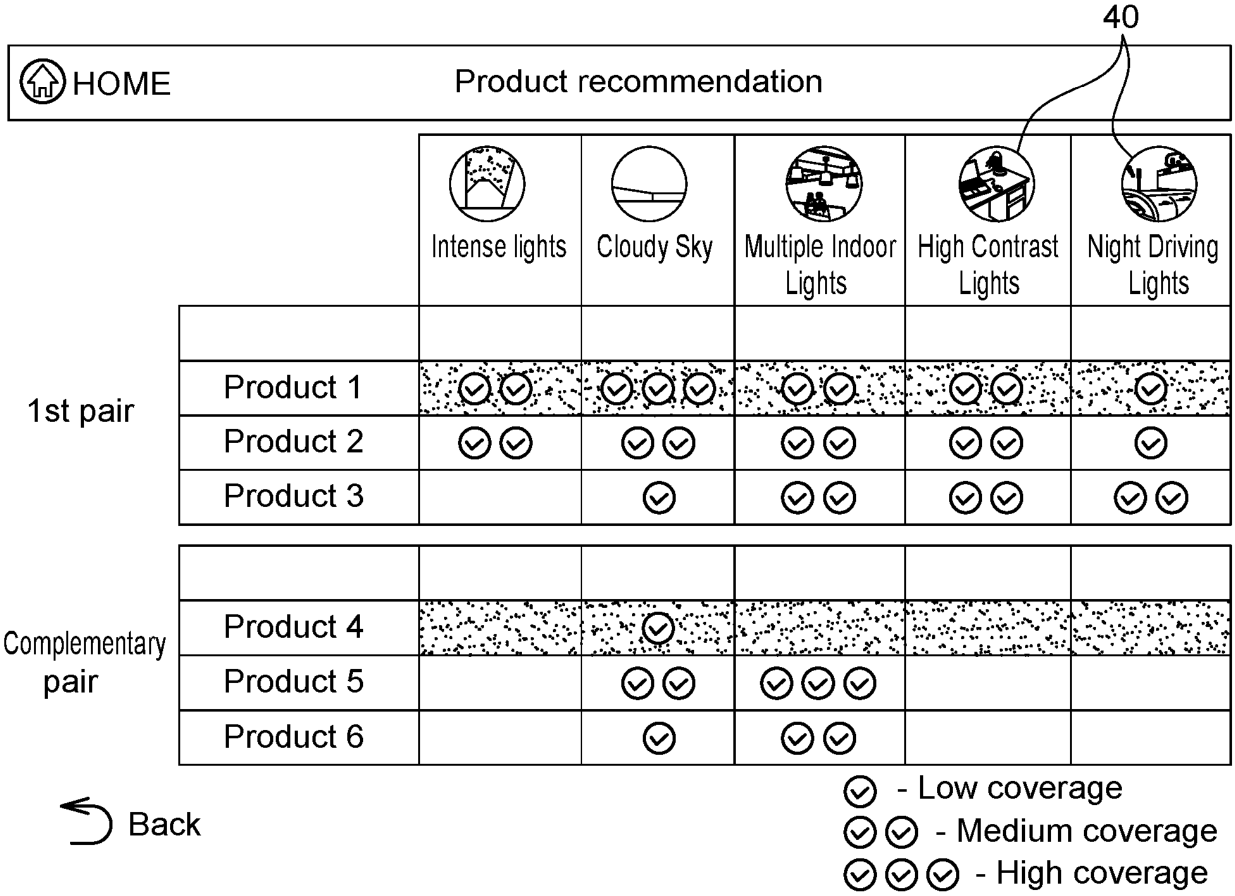
40
HOME
Product recommendation
Intense lights
Cloudy Sky
Multiple Indoor Lights
High Contrast Lights
Night Driving Lights
1st pair
Product 1
Product 2
Product 3
Complementary pair
Product 4
Product 5
Product 6
Back
- Low coverage
- Medium coverage
- High coverage

Fig. 12

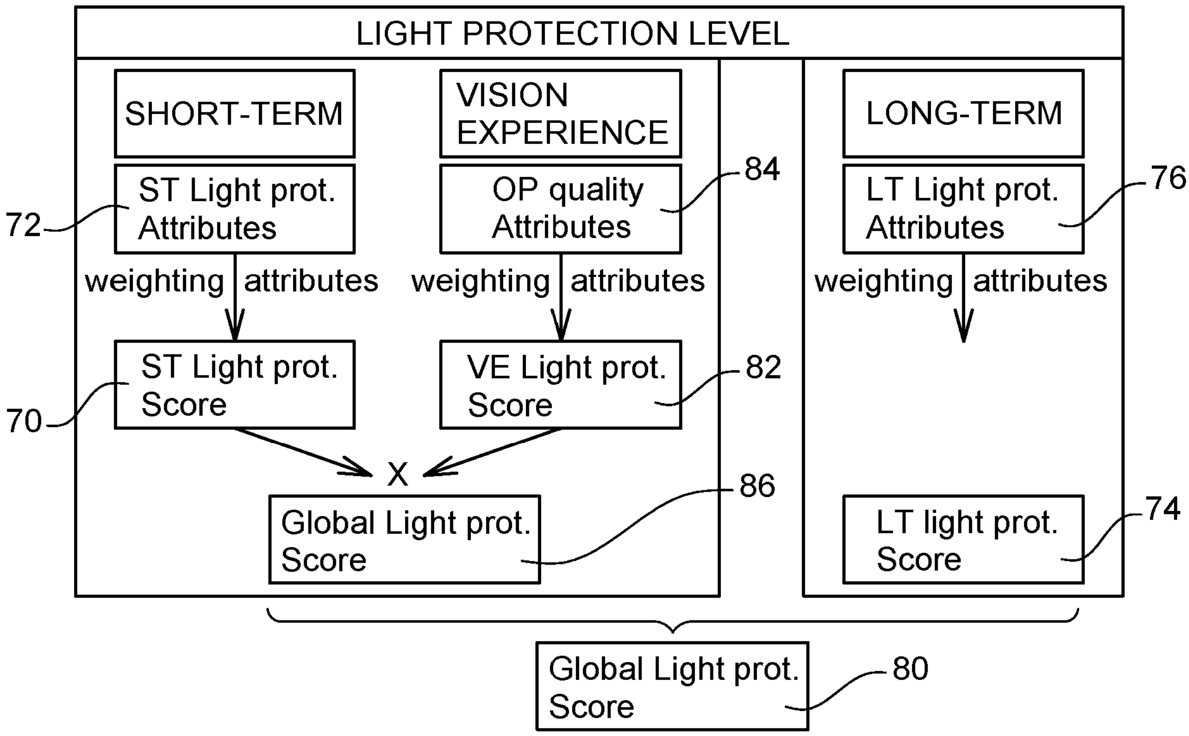
LIGHT PROTECTION LEVEL
SHORT-TERM
VISION EXPERIENCE
LONG-TERM
ST Light prot. Attributes
OP quality Attributes
LT Light prot. Attributes
weighting attributes
weighting attributes
weighting attributes
ST Light prot. Score
VE Light prot. Score
X
Global Light prot. Score
LT light prot. Score
Global Light prot. Score
72
70
84
82
86
76
74
80

# Fig. 13

| | | Weighting factors | | | |
|---|---|---|---|---|---|
| | | $X_1$ | $X_2$ | $X_3$ | $X_4$ |
| QUALITY | OPQ Att.1 | $\geqslant V_1$ | $\leqslant V_2$ | $\leqslant V_3$ | $\leqslant V_4$ |
| | OPQ Att.2 | ⋮ | ⋮ | ⋮ | ⋮ |
| | OPQ Att.3 | | | | |
| | OPQ Att.4 | | | | |
| | OPQ Att.5 | | | | |
| | OPQ Att.6 | | | | |

# Fig. 14

| LIGHT SITUATION | LIGHT PROTECTION ATTRIBUTES | | OP QUALITY ATTRIBUTES |
|---|---|---|---|
| | Short-term | Long-term | |
| Bright light | ST Att.1,ST Att.2 | LT Att.1,LT Att.2 | OPQ Att.1,OPQ Att.2,OPQ Att.3 |
| Day drive | ST Att.3,ST Att.4 | LT Att.3,LT Att.4 | OPQ Att.4,OPQ Att.5,OPQ Att.6 |
| Night drive | ST Att.5,ST Att.6 | LT Att.5,LT Att.6 | OPQ Att.7,OPQ Att.8,OPQ Att.9 |
| Indoor | ST Att.7,ST Att.8 | LT Att.7,LT Att.8 | OPQ Att.10,OPQ Att.11,OPQ Att.12 |
| Screen at night | ST Att.9,ST Att.10 | LT Att.9,LT Att.10 | OPQ Att.13,OPQ Att.14,OPQ Att.15 |
| Transitions In-Out | ST Att.11,ST Att.12 | LT Att.11,LT Att.12 | OPQ Att.16,OPQ Att.17,OPQ Att.18 |
| Transitions Out-In | ST Att.13,ST Att.14 | LT Att.13,LT Att.14 | OPQ Att.19,OPQ Att.20,OPQ Att.21 |

# Fig. 15

| LIGHT SITUATION | LIGHT PROTECTION ATTRIBUTES | | | | OPTICAL PRODUCT QUALITY ATTRIBUTES | | |
|---|---|---|---|---|---|---|---|
| | Short-term | | Long-term | | | | |
| Bright light | ST Att.1 | ST Att.2 | LT Att.1 | LT Att.2 | OPQ Att.1 | OPQ Att.2 | OPQ Att.3 |
| | x % | x % | x % | x % | x % | x % | x % |
| Day drive | ST Att.3 | ST Att.4 | LT Att.3 | LT Att.4 | OPQ Att.4 | OPQ Att.5 | OPQ Att.6 |
| | x % | x % | x % | x % | x % | x % | x % |
| Night drive | ST Att.5 | ST Att.6 | LT Att.5 | LT Att.6 | OPQ Att.7 | OPQ Att.8 | OPQ Att.9 |
| | x % | x % | x % | x % | x % | x % | x % |
| Indoor | ST Att.7 | ST Att.8 | LT Att.7 | LT Att.8 | OPQ Att.10 | OPQ Att.11 | OPQ Att.12 |
| | x % | x % | x % | x % | x % | x % | x % |
| Screen at night | ST Att.9 | ST Att.10 | LT Att.9 | LT Att.10 | OPQ Att.13 | OPQ Att.14 | OPQ Att.15 |
| | x % | x % | x % | x % | x % | x % | x % |
| Transitions In-Out | ST Att.11 | ST Att.12 | LT Att.11 | LT Att.12 | OPQ Att.16 | OPQ Att.17 | OPQ Att.18 |
| | x % | x % | x % | x % | x % | x % | x % |
| Transitions Out-In | ST Att.13 | ST Att.14 | LT Att.13 | LT Att.14 | OPQ Att.19 | OPQ Att.20 | OPQ Att.21 |
| | x % | x % | x % | x % | x % | x % | x % |

# Fig. 16

| | DATA | OP1 | OP2 | OP3 | OP4 | OP5 | OP6 | OP7 | OP8 | OP9 | OP10 | OP11 | OP12 | OP13 | OP14 | OP15 |
|---|---|---|---|---|---|---|---|---|---|---|---|---|---|---|---|---|
| 72, 76 | Tv(23+35 average) | 19 | 20.75 | 18.6 | 14 | 89.13 | 97.35 | 88.2 | 94.48 | 87.93 | 96.6 | 96.1 | 15.36 | 17.45 | 18.13 | 15.57 |
| | PE | 0 | 0 | 90 | 0 | 0 | 0 | 0 | 0 | 0 | 0 | 0 | 0 | 0 | 99 | 99 |
| | BVC | 87 | 86 | 90 | 87 | 14.49 | 8.65 | 27.3 | 24.03 | 31 | 22 | 25.4 | 87.68 | 86.79 | 81.8 | 84.75 |
| | E-SPF | 16 | 40 | 46 | 43 | 15 | 35 | 15 | 52 | 15 | 50 | 50 | 16 | 100 | 100 | 82 |
| 84 | Rv | 5.18 | 0.69 | 0.64 | 0.68 | 5.39 | 0.8 | >5 | 0.65 | 5.36 | 0.65 | | 5.36 | 1.18 | 1.38 | 1.42 |
| | Haze | 0.15 | 0.14 | 0.58 | 0.16 | 0.09 | 0.01 | | 0.09 | 0.12 | 0.1 | | 0.08 | 0.11 | 0.1 | 0.14 |
| | b* | 6.62 | 4.45 | 7.47 | 8.06 | 0.82 | 2.22 | 28 | 3.95 | 3.49 | 2.9 | 4.8 | -10.87 | -9.37 | -1.9 | -5.34 |
| | | | | | | | | | | | | | | | | |
| | SCORING ATTRIBUTES | | | | | | | | | | | | | | | |
| 70 | Tv | 2.82 | 2.58 | 2.88 | 3.62 | 0.08 | 0.06 | 0.09 | 0.06 | 0.09 | 0.06 | 0.06 | 3.38 | 3.04 | 2.94 | 3.35 |
| | PE | 0 | 0 | 4 | 0 | 0 | 0 | 0 | 0 | 0 | 0 | 0 | 0 | 0 | 4 | 4 |
| 74 | BVC | 4 | 4 | 4 | 4 | 1 | 0 | 2 | 2 | 2 | 2 | 2 | 4 | 4 | 4 | 4 |
| | E-SPF | 2 | 3 | 3 | 3 | 1 | 3 | 1 | 4 | 1 | 4 | 4 | 1 | 4 | 4 | 4 |
| 82 | Rv | 0.5 | 1 | 1 | 1 | 0.5 | 1 | 0.5 | 1 | 0.5 | 1 | 1 | 0.5 | 0.9 | 0.9 | 0.9 |
| | Haze | 1 | 1 | 1 | 1 | 1 | 1 | 1 | 1 | 1 | 1 | 1 | 1 | 1 | 1 | 1 |
| | b* | 0.9 | 1 | 0.9 | 0.9 | 1 | 1 | 1 | 1 | 1 | 1 | | | | | |

# Fig. 17

| | | OP1 | OP2 | OP3 | OP4 | OP5 | OP6 | OP7 | OP8 | OP9 | OP10 | OP11 | OP12 | OP13 | OP14 | OP15 |
|---|---|---|---|---|---|---|---|---|---|---|---|---|---|---|---|---|
| 70 | SHORT | 0.5 | 0.5 | 0.8 | 0.7 | 0.0 | 0.0 | 0.0 | 0.0 | 0.0 | 0.0 | 0.0 | 0.6 | 0.6 | 0.8 | 0.9 |
| 74 | LONG | 0.8 | 0.9 | 0.9 | 0.9 | 0.3 | 0.4 | 0.4 | 0.8 | 0.4 | 0.8 | 0.8 | 0.6 | 1.0 | 1.0 | 1.0 |
| 82 | Vision experience factor | 0.75 | 1 | 1 | 1 | 0.83 | 1.00 | 0.83 | 1.00 | 0.83 | 0.9 | 0.9 | 0.75 | 0.95 | 0.95 | 0.95 |
| 86 | ST * VE | 0.40 | 0.48 | 0.79 | 0.68 | 0.01 | 0.01 | 0.01 | 0.01 | 0.01 | 0.01 | 0.01 | 0.48 | 0.54 | 0.76 | 0.83 |
| 80 | LPS(ST+VE)*LT | 0.57 | 0.68 | 0.83 | 0.78 | 0.13 | 0.19 | 0.19 | 0.38 | 0.19 | 0.38 | 0.38 | 0.55 | 0.77 | 1.88 | 0.92 |

# Fig. 18

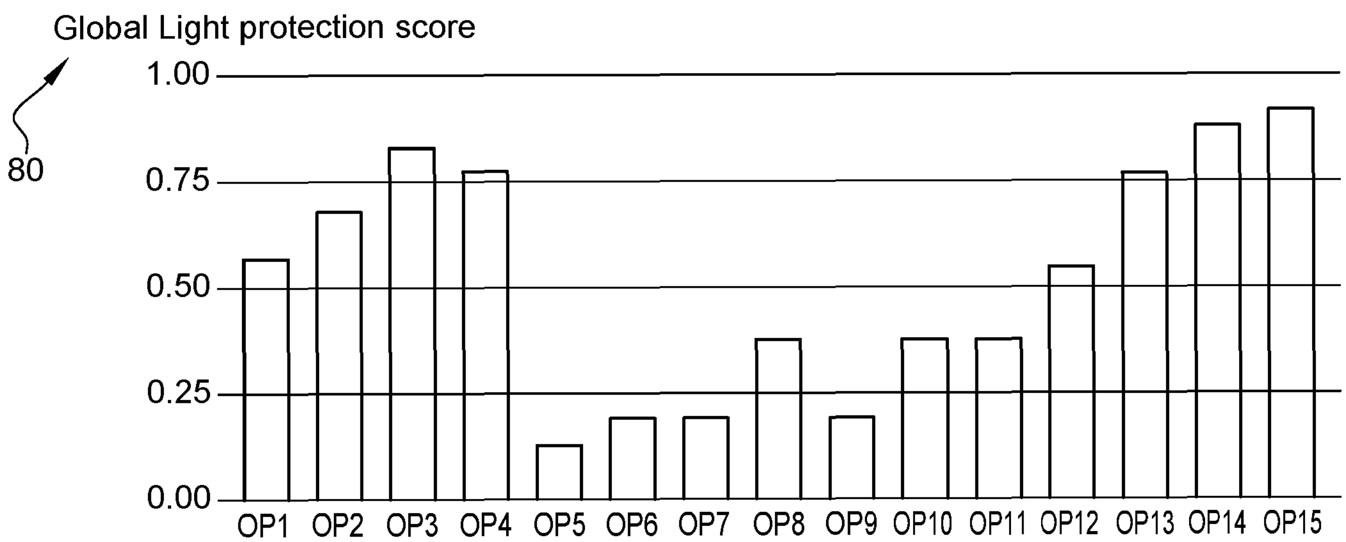

Fig. 19

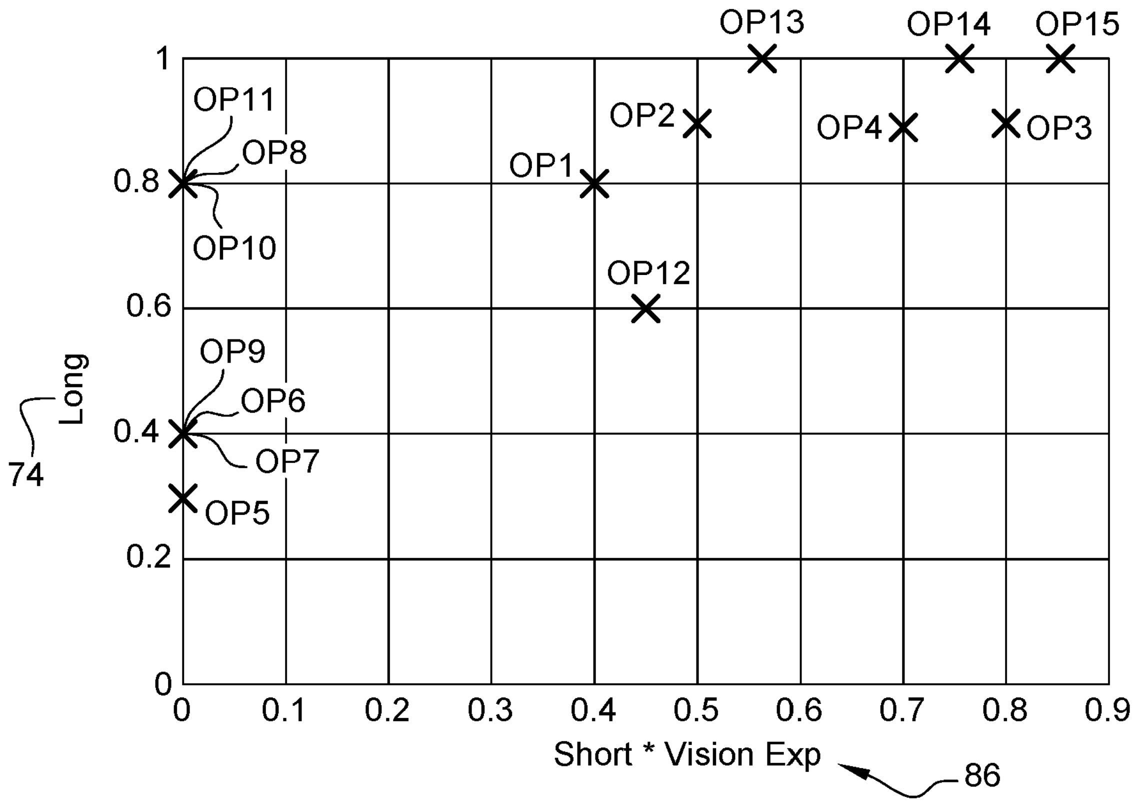
OP13
OP14
OP15
1
OP11
OP2
OP4
OP3
OP8
0.8
OP1
OP10
OP12
0.6
OP9
Long
OP6
74
0.4
OP7
OP5
0.2
0
0
0.1
0.2
0.3
0.4
0.5
0.6
0.7
0.8
0.9
Short * Vision Exp
86

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3753475 A
- US 6099126 A
- US 2019212581 A
- US 4689387 A
- US 4775733 A
- US 5059673 A
- US 5087758 A
- US 5191055 A
- US 5645767 A
- US 5658501 A
- US 4931220 A
- US 20030174560
- WO 2012076714 A
- EP 2607884 A
- EP 20306264

### Non-patent literature cited in the description


- **MAINSTER MA** ; **TURNER PL**. Glare's causes, consequences, and clinical challenges after a century of ophthalmic study. *American journal of ophthalmology*, 2012, vol. 153 (4), 587-593
- **EFALOV VJ**. Rod and cone visual pigments and phototransduction through pharmacological, genetic, and physiological approaches. *J Biol Chem.*, 2012, vol. 287 (3), 1635-1641
- **NOSEDA R** ; **KAINZ V** ; **JAKUBOWSKI M et al.** A neural mechanism for exacerbation of headache by light. *Nat Neurosci.*, 2010, vol. 13 (2), 239-245
- **MARIE et al.** *Cell Death and Disease*, 2020
- **MARIE et al.** *Cell Death and Disease*, 2018
- **ARNAULT et al.** *PlosOne*, 2013
- **ALEXANDER KOKKA et al.** *Metrologia*, 2018, vol. 55, 526